(19) Europäisches Patentamt

European Patent Office

Office européen des brevets

(11) **EP 2 362 768 B1**

(12) **EUROPEAN PATENT SPECIFICATION**

(45) Date of publication and mention
of the grant of the patent:
**20.04.2016 Bulletin 2016/16**

(51) Int Cl.:
*A61K 9/20* (2006.01)          *A61K 9/48* (2006.01)
*A61K 9/00* (2006.01)          *A61K 31/167* (2006.01)
*A61K 31/485* (2006.01)

(21) Application number: **09740619.3**

(22) Date of filing: **23.10.2009**

(86) International application number:
**PCT/US2009/061803**

(87) International publication number:
**WO 2010/062524 (03.06.2010 Gazette 2010/22)**

(54) **EXTENDED RELEASE ORAL ACETAMINOPHEN/TRAMADOL DOSAGE FORM**

ORALE DOSIERFORM VON ACETAMINOPHEN/TRAMADOL MIT VERLÄNGERTER FREISETZUNG

FORME POSOLOGIQUE ORALE D'ACÉTAMINOPHÈNE/TRAMADOL À LIBÉRATION PROLONGÉE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **27.10.2008 US 108618 P**

(43) Date of publication of application:
**07.09.2011 Bulletin 2011/36**

(73) Proprietor: **Alza Corporation
Vacaville, CA 95688 (US)**

(72) Inventors:
• **DAI, Wei-guo
Devon, PA 19333 (US)**
• **DONG, Liang-chang
Sunnyvale
CA 94086 (US)**

• **CHOI, Tae-hong
Kyunggi-do (KR)**
• **HWANG, Sung Joo
Seoul 100-138 (KR)**
• **KIM, Jae, Hyun
Seoul 100-138 (KR)**
• **LEE, Dong, Ho
Seoul (KR)**

(74) Representative: **Warner, James Alexander
Carpmaels & Ransford LLP
One Southampton Row
London WC1B 5HA (GB)**

(56) References cited:
**WO-A-2006/053012          WO-A-2007/048219
WO-A-2008/064854          WO-A-2009/114648
US-A1- 2004 131 671        US-B1- 6 375 957**

**Description**

TECHNICAL FIELD

This invention relates to extended release of drugs. In particular, the invention relates to extended release dosage forms of a combination of acetaminophen and tramadol.

BACKGROUND

Chronic pain, such as lower back pain and osteoarthritis flare pain, is a major health issue that causes severe personal suffering, great loss in economic productivity, as well as tremendous direct and indirect cost to society as a whole. Approximately 60% to 80% of adults in United States are estimated to suffer the chronic lower back pain sometime in their life. Presently, with aging population in many countries, chronic pain is a growing concern. Nonsteroidal anti-inflammatory drugs (NSAIDs) are commonly used for treatments of chronic pain, but with limited efficacy. Moreover, NSAIDs are often associated with substantial health risk, including gastrointestinal lesion, ulceration, bleeding, even death. Therefore, there is a medical need for improved treatments for these chronic pains.

Tramadol, (2-(dimethylaminomethyl)-1-(3-methoxyphenyl)-cyclohexan-1-ol, $C_{16}H_{25}NO_2$), is a centrally acting analgesic, whereas NSAIDs are the peripherally acting ones. The tramadol's mode of action is not completely understood; but in-vivo result suggests dual mechanisms: binding of the parent molecule and its metabolite to mu-opioid receptors and weak inhibition of reuptake of norepinephrine and serotonin. Acetaminophen, (N-(4-hydroxyphenyl) acetamide, $C_8H_9NO_2$) (or "APAP"), e.g., the commonly known TYLENOL brand, has been a first-choice analgesic for the treatment of chronic pain for many years. Although the action mechanism of APAP remains uncertain, it appears to be also centrally mediated, involving selective inhibition of prostaglandin synthesis in the CNS, inhibition of *N*-methyl-D-aspartate or substance P-mediated nitric oxide synthesis and inhibition of prostaglandin-E2 release in the spinal cord.

Tramadol and APAP have been combined in delivery. US patent RE39221 describes that the combination employs lesser amounts of both the tramadol material and APAP than would be necessary to produce the same amount of analgesia if either was used alone. Ortho-McNeil Pharmaceutical developed a proprietary oral immediate-released dosage form of tramadol/APAP (37.5/325 mg) combination (ULTRACET), which was approved by the FDA in 2001 for management of acute pain. This product shows no side effects associated with the use of NSAIDs, such as gastrointestinal ulcers or bleeding. In addition, clinical trials have demonstrated synergistic effect of the combination, which provides longer action duration than APAP and a faster onset of action than tramadol. For ULTRACET, doses have to be taken every 4 to 6 hours.

Acetaminophen (or APAP herein) (Mw 151.163 g/mol) and tramadol (can be referred to as TRD herein) (Mw 263.375 g/mol) are weak bases with p*Ka* values of 9.38 and 9.41, respectively. Aqueous solubility of APAP is about 14 mg/ml, while tramadol HCl is freely soluble in water. After oral administration, APAP and tramadol HCl are rapidly absorbed, and both drugs undergo significant first-pass metabolism. Although absorption of APAP following administration of drug dosage forms occurs primarily in the small intestine, it also appears to have good colonic absorption. The extended release (ER) oral dosage form of APAP (TYLENOL® ER, by McNeil Consumer Healthcare) became commercially available in 1995. This bi-layer matrix tablet is composed of 325 mg of APAP in the immediate release layer and additional 325 mg of APAP in the extended release layer. The Extended release of APAP is achieved by controlling drug diffusion in the hydrophilic polymer matrix.

Regarding tramadol, bioavailability of current extended release dosage forms of tramadol HCl, ULTRAM® ER and tramadol HCl CONTRAMID® OAD, implies an acceptable absorption in the low gastrointestinal tract. These two products provide with effective pain control over a 24-hour period in a convenient once-daily form. The ULTRAM® ER product has a core coated with a mixture of a semipermeable polymer and a water-soluble permeation enhancer. A graduated release of tramadol HCl from the tablet is achieved by controlling the coating membranes. CONTRAMID® OAD is a compress-coated matrix tablet. The core matrix is the crosslinked high amylase starch, which provides with slow release, while the compressed coat imparts the relatively faster release.

However, there are technical challenges in developing the extended release dosage form for APAP/tramadol HCl combination, using either hydrophilic polymer matrix approach as that for TYLENOL® ER or the coated tablet approach as that for ULTRAM® ER and CONTRAMID® OAD. An undesirable drug burst with hydrophilic matrix system is often observed for highly water-soluble drugs like tramadol HCl, due to rapid diffusion of the dissolved drug through the hydrophilic gel network. Also, the large difference in water solubility of the two drugs makes the use of coating to provide extended release impractical to achieve a synchronized release of both APAP and tramadol HCl. Attempts have been made to provide extended release of APAP and tramadol, e.g., WO2004026308 and US patent publication US20040131671. However, well-coordinated release is hard to achieve. What is needed is an extended release dosage form of tramadol and APAP that can deliver synchronized (or coordinated) release of the two drugs for an extended period of time in that the cumulative weight percent release of the two drug are not very different.

SUMMARY

The present invention provides a method and a dosage form having APAP and tramadol for extended delivery. In the dosage form of the present invention, the drug/polymer ionic interaction between tramadol and an anionic polymer provides a slow release of tramadol to result in a coordinated release of APAP and tramadol.

In one aspect, the present invention provides a pharmaceutical composition containing APAP and a complex tramadol material that the composition exhibits coordinated sustained release upon dissolution as in oral administration in a patient, resulting in coordinated accumulative (i.e., cumulative) release of tramadol and accumulated release of APAP over time. The composition can be a tablet or a part of a tablet, which when in the gastrointestinal tract slowly disintegrates to release tramadol and APAP in a coordinated release profile. The composition includes complexed tramadol material, and the complexation is done using carrageenan. The tramadol is preferably a tramadol salt, more preferably a hydrochloride (HCl) salt.

In another aspect, the composition containing the complexed tramadol material and APAP results in the sustained release for a period of 4 to 12 hours, and especially from over 6 hours to 12 hours, over the whole period of sustained delivery for which the dosage form is designed for both tramadol and APAP. It is to be noted that when a drug is approved by a competent regulatory authority (e.g., USFDA) for treating patients, the dosage form is approved for a dose to be taken periodically, at dose period intervals. Thus, the application and approval for a dosage form specifies such dose periods for which the dosage form is designed.

In one aspect, the invention provides a method of making a dose form of a pharmaceutical composition, in which the method includes the steps of forming a complex tramadol material and forming a compacted form including the complex tramadol material and APAP. The compacted form exhibits coordinated sustained release upon oral administration in a patient resulting in coordinated accumulative release of tramadol and accumulated release of APAP over time. The composition can be a tablet or part (such as a layer) of a tablet, which provides sustained, coordinated extended release (ER) of tramadol and APAP. In one aspect, a dosage form can be a bi-layer tablet in which two layers are attached together one on the other: one extended release (ER) layer containing APAP and a tramadol complex and an immediate release (IR) layer containing APAP and a noncomplexed tramadol. In another aspect, a dosage form can includes an ER material containing APAP and a tramadol complex surrounded on all sides or sandwiched on both sides by an IR layer of APAP and a noncomplexed tramadol.

In one aspect, the invention provides of using a complex tramadol material in the manufacture of a medicament for the treatment of pain, and a complex tramadol material for use in a method of treating pain with the medicament. The medicament contains a complex tramadol material and APAP, the medicament exhibits coordinated sustained release of the tramadol and APAP upon dissolution as in oral administration of the medicament in a patient resulting in coordinated cumulative release of tramadol and cumulated release of APAP over time.

We have found that certain anionic polymers, especially carrageenans, decrease the drug solubility and diffusivity or dissolution, leading to a sustained, extended release of tramadol. Thus, the combination of APAP with tramadol complexed with carrageenan produced sustained release of tramadol that matches closely with the release profile of APAP in terms of percentage of cumulative release of the drugs. This coordinated delivery of the two drugs in an extended period of time offers significant advantage over previously available dosage forms that require frequent dosing and large fluctuation of plasma concentration of APAP and tramadol. The release of drugs from a sustained or extended release formulation depends on the controlled release of two different drugs, one of which is usually faster than the other if uncontrolled. It is unpredictable that a drug that is released quickly can be delayed in release to match the release of a relatively slow releasing drug. Thus, it is surprising that the use of selected anionic complexing polymer, especially carrageenan, enables us to achieve extended release in which the releases of APAP and tramadol are closed matched. We found that the complexation can modify the release kinetics, from Fickian ($n$= about 0.45 in Korsmeyer equation) to more zero order release ($n$ approaching to 1 in Korsmeyer equation) and slow down the release rate as well. Therefore, complexing tramadol with carrageenan, especially lambda carrageenan will reduce the release rate gap for tramadol and APAP, thereby synchronizing their release rates. The formulation can preferably contain two other excipients, PEO and HPMC K4M as release retarding agent with complexing agent, carrageenan. Without PEO, the complex mixture is harder to compress into tablets due to lamination and/or capping, and hard to achieve the proper hardness when compressed. Also, the compressed ER tablet without PEO showed less zero-order kinetics characteristics in dissolution than those with PEO even if the drugs are synchronized with carrageenan complexation. Therefore, PEO helps to provide release kinetics of APAP and tramadol that is near zero-order and also to provide better compressibility and manufacturability. It has also been found that HPMC K4M contributed to the tablet compressibility and improved a sustained release of both APAP and tramadol.

BRIEF DESCRIPTION OF THE DRAWINGS

FIG. 1A illustrates a sectional view in portion of a bi-layer tablet dosage form of APAP and tramadol according to the present invention.

FIG. 1B illustrates a cross-section view through another embodiment of a tablet dosage form of APAP and tramadol in which an ER layer is surrounded by IR layer according to the present invention.

FIG. 1C illustrates a cross-section view through another embodiment of a tablet dosage form of APAP and tramadol according to the present invention, in which an ER layer is sandwiched between layers of IR material.

FIG. 2 shows the release profile of APAP/tramadol combination from a matrix in which the tramadol is complexed and one that is not complexed.

FIGs. 3, 4 and 5 show the release profiles for Formulations C, D and E, respectively, having different amounts of hydroxypropylmethyl cellulose K4M (HPMC K4M).

FIG. 6 shows the $T_{80}$ for APAP and the duration ratio for the tramadol to illustrate the effect of HPMC.

FIGs 7a and 7b show the release profiles of tramadol and APAP for composition F and G, respectively, illustrating the effect of having and not having a complex of tramadol and carrageenan.

FIG. 8 is graphical release profile of APAP for 4 formulations F-No.2 to F-No. 5 having fillers such as lactose, AEROSIL and polyethylene oxide.

FIG. 9 is graphical release profile of tramadol HCl for the 4 formulations F-No.2 to F-No. 5 of FIG. 8 having fillers such as lactose, AEROSIL and polyethylene oxide.

FIG. 10 is graphical release profile of APAP for the 4 formulations F-No.2 to F-No. 5 of FIG. 8 having fillers such as lactose, AEROSIL and polyethylene oxide with the assumption of a bi-layer dosage form of IR and ER.

FIG. 11 is graphical release profile of tramadol HCl for the 4 formulations F-No.2 to F-No. 5 of FIG. 8 having fillers such as lactose, AEROSIL and polyethylene oxide with the assumption of a bi-layer dosage form of IR and ER.

FIG. 12 is graphical release profile of APAP and tramadol HCl from formulation F-No. 6.

FIG. 13 is graphical release profile of APAP and tramadol HCl from formulation F-No. 6 with the assumption of a bi-layer dosage form of IR and ER.

FIG. 14 is graphical release profile of APAP from formulation F-No. 7 and Formulation F-No. 8.

FIG. 15 is graphical release profile of tramadol HCl from formulation F-No. 7 and Formulation F-No. 8.

FIG. 16 is graphical release profile of APAP from formulation F-No. 7 and Formulation F-No. 8 with the assumption of a bi-layer dosage form of IR and ER.

FIG. 17 is graphical release profile of tramadol HCl from formulation F-No. 7 and formulation F-No. 8 with the assumption of a bi-layer dosage form of IR and ER.

FIG. 18 is graphical release profile of APAP from formulation F-No. 7, formulation F-No. 9, and formulation F-No. 10.

FIG. 19 is graphical release profile of tramadol HCl from formulation F-No. 7, formulation F-No. 9, and formulation F-No. 10.

FIG. 20 is graphical release profile of APAP from formulation F-No. 7, formulation F-No. 9, and formulation F-No. 10 with the assumption of a bi-layer dosage form of IR and ER.

FIG. 21 is graphical release profile of tramadol HCl from formulation F-No. 7, formulation F-No. 9, and formulation F-No. 10 with the assumption of a bi-layer dosage form of IR and ER.

FIG. 22 is graphical release profile of APAP from formulation F-No. 10, formulation F-No. 11, and formulation F-No. 12.

FIG. 23 is graphical release profile of tramadol HCl from formulation F-No. 10, formulation F-No. 11, and formulation F-No. 12.

FIG. 24 is graphical release profile of APAP from formulation F-No. 10, formulation F-No. 11, and formulation F-No. 12 with the assumption of a bi-layer dosage form of IR and ER.

FIG. 25 is graphical release profile of tramadol HCl from formulation F-No. 10, formulation F-No. 11, and formulation F-No. 12 with the assumption of a bi-layer dosage form of IR and ER.

FIG. 26 is graphical release profile of APAP from formulation F-No. 10 in buffers of different pH and distilled water.

FIG. 27 is graphical release profile of tramadol HCl from formulation F-No. 10 in buffers of different pH and distilled water.

FIG. 28 is graphical release profile of APAP from formulation F-No. 10 in buffers of different pH and distilled water with the assumption of a bi-layer dosage form of IR and ER.

FIG. 29 is graphical release profile of tramadol HCl from formulation F-No. 10 in buffers of different pH and distilled water with the assumption of a bi-layer dosage form of IR and ER.

FIG. 30 is graphical release profile of APAP from formulation F-No. 7 at different speed (rpm) of stirring in dissolution.

FIG. 31 is graphical release profile of tramadol HCl from formulation F-No. 7 at different speed (rpm) of stirring in dissolution.

FIG. 32 is graphical release profile of APAP from formulation F-No. 7 at different speed (rpm) of stirring in dissolution with the assumption of a bi-layer dosage form of IR and ER.

FIG. 33 is graphical release profile of tramadol HCl from formulation F-No. 7 at different speed (rpm) of stirring in dissolution with the assumption of a bi-layer dosage form of IR and ER

FIG. 34 shows dissolution profiles for the F-No. 13 for (a) APAP and (b) tramadol HCl from F-No. 13 at 50 rpm in pH 1.2 buffer for the first 2 hours and pH 6.8 buffer from 2 to 12 hours.

FIG. 35 shows a flow chart for the manufacturing process for making the bi-layer tablet embodiment of F-No. 13.

FIG. 36 shows a graphical representation in portion the mean plasma concentration-time profiles of tramadol after multiple oral administrations of ULTRACET tablets and ER tablets of the present invention

FIG. 37 shows a graphical representation in portion of the mean plasma concentration-time profiles of APAP after multiple oral administrations of ULTRACET tablets and ER tablets of the present invention

DETAILED DESCRIPTION

The present invention relates to a dosage form that delivers coordinated delivery of APAP and tramadol to a patient through oral administration. More specifically the present invention relates to a dosage form that delivers coordinated delivery of APAP and tramadol to a patient via the gastrointestinal tract in extended delivery during which the dosage form disintegrates and the drugs are released gradually over an extended period of time.

In describing the present invention, the following terms will be employed, and are intended to be defined as indicated below. As used in this specification and the appended claims, the singular forms "a," "an" and "the" include plural references unless the text content clearly dictates otherwise.

As used herein, the term "tramadol", unless specified otherwise in the content, can mean tramadol base, tramadol salt or a tramadol derivative that have cationic property to complex with carrageenan by ionic interaction. The amount of tramadol mentioned herein refers to tramadol HCl equivalent.

"Biologically active agent" is to be construed in its broadest sense to mean any material that is intended to produce some biological, beneficial, therapeutic, or other intended effect, such as enhancing permeation, relief of pain and contraception. As used herein, the term "drug" refers to any material that is intended to produce some biological, beneficial, therapeutic, or other intended effect.

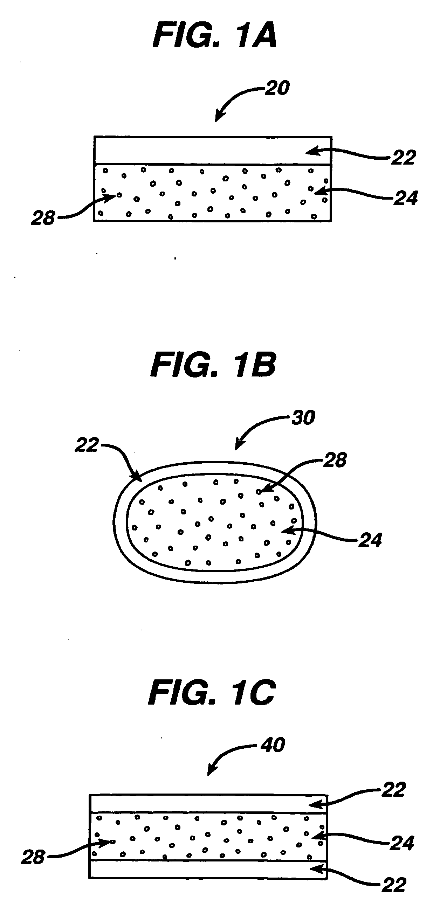

FIG. 1A is a schematic, cross-sectional artist's rendition of a bi-layer tablet, i.e., a tablet having two layers. In a bi-layer tablet, the two layers can be in direct and intimate contact, such as where one layer is on top of another layer. In an embodiment, the tablet 20 includes an extended release (ER) layer 24 (which includes tramadol complex particles 28) connected together with an immediate release (IR) layer 22. The dosage form has only two layers with active pharmaceutical ingredients (APIs) (APAP and tramadol). In another embodiment, the structure shown in FIG. 1A can be a portion of whole cross section of a form shown in FIG. 1B. The form can be a traditional pill shape, elongated tablet shape, spherical shape, cucumber shape, etc., which for convenience herein are referred to as "tablet", unless the word "tablet" is specified to be otherwise with specificity. In the form shown in FIG. 1B, the tablet 30 includes an extended release (ER) layer 24 (which includes tramadol complex particles 28) surrounded by an immediate release (IR) layer 22. Thus, the ER material can be a core (preferably layer-shaped or tablet-shaped) surrounded by an IR layer. Further, the tablet can have an ER layer sandwiched between two IR layers, as tablet 40 shown in FIG. 1C. The tablet of any form may additionally include an outer coating (or coat, although not shown in the FIGs. 1A-1C). The outer coating can surround the IR layer 22 and any ER material that is not surrounded by the IR layer.

In one aspect, a dosage form of the present invention includes a solid, compacted form that releases APAP and tramadol slowly over a period of time in extended release. For example, the solid, compacted dosage form can be one layer of a bi-layer tablet or as core that is surrounded by a fast release (or immediate release) outer layer. Generally, the solid compacted form includes a complexed tramadol material that slowly releases tramadol active moiety into the gastrointestinal tract and is absorbed. Complex formation of carrageenan with a basic drug is described in Aguzzi et al., "Influence of Complex solubility on Formulations based on Lambda Carrageenan and Basic Drugs", AAPS PharmSciTech 2002; 3(3) Article 27.

The complex tramadol material includes a tramadol material, which can be tramadol base or a salt or ester thereof. The tramadol material is any one of (1R, 2R or 1S, 2S)-(dimethylaminomethyl)-1-(3-methoxyphenyl)-cyclohexanol (tramadol), its N-oxide derivative ("tramadol N-oxide"), and its O-desmethyl derivative ("O-desmethyl tramadol") or mixtures thereof. It also includes the individual stereoisomers, mixtures of stereoisomers, including the racemates, pharmaceutically acceptable salts of the amines, such as the hydrochloride salt, citrate, acetate, solvates and polymorphs of the tramadol material. Tramadol is commercially available from Grunenthal. Methods of making tramadol are known in the art, e.g., as described in U.S. Pat. No. 3,652,589 and RE39221, which are herein incorporated by reference. O-Desmethyl tramadol is prepared by treating tramadol as a free base under O-desmethylating reaction conditions, e.g., reacting it with a strong base such as NaOH or KOH, thiophenol and diethylene glycol (DEG) with heating to reflux. See, Wildes et al., J. Org. Chem., 36, 721 (1971). Tramadol HCl is preferred as the tramadol material for complexing with the anionic polymer. It is contemplated that the use of tramadol base or different salts in association with tramadol, such as different halogen salts, etc., of tramadol will not affect much the complex formation of the tramadol with carrageenan and therefore will not result in a significant difference in the release rate of the resulting ER tablet. One skilled in the art will be able to adjust the formulation accordingly based on the present description without undue experimentation.

Complexing polymers are water soluble, gel forming and anionic; they contain pendant groups such as sulfate, carboxylate, phosphate or other negatively charged groups to interact with the cationic drug. Preferably, the complexing polymer is a polysaccharide-based material with pendant anionic groups (in other words, anionic polysaccharide, especially sulfated polysaccharide). Especially preferred is carrageenan. Carrageenans are sulfated polysaccharides obtained from seaweeds. Generally the types of carrageenans include kappa, iota, and lambda, all of which form gels with water at room temperature. Different types of carrageenans might form gels of different softness or toughness characteristics. The complexing of λ-carrageenan with basic drugs has been described by Aguzzi et al. (AAPS PharmSciTech 2002; 3(3) Article 27), incorporated by reference herein.

The complexing polymers are biocompatible and non-toxic. They are of sufficiently high molecular weight that a gel can be prepared with the active agent. While not wishing to be bound to a particular theory, it is believed that the cationic drug interacts with the anionic pendant groups of the anionic polymer and causing the electrostatic interactions between polymer strands, causing the polymer strands to be positioned in such a way to slow the penetration of polar solvent (e.g., water) to the tramadol. Generally, the MW of lambda carrageenan is between 100,000~ 500,000 Daltons. Lambda carrageenan is commercially available as two kinds by viscosity. One is VISCARIN® GP 109 from FMC (low viscosity, having a viscosity of about 760 cPs measured at 37° C with a shear rate of 20 s$^{-1}$) and another is VISCARIN® GP 209 (high density, having a viscosity of about 1600 cPs measured at 37° C with a shear rate of 20 s$^{-1}$). In this study, it has been found that VISCARIN® GP 109 was more useful. The preferred grade of carrageenan is low molecular weight of lambda carrageenan. Other carrageenans, such as kappa-carrageen can also be used. Lambda carrageenan is characterized by the highest amount of sulfate groups in comparison with the analogous kappa and iota types. It has been demonstrated that lambda carrageenan can interact strongly with very soluble drugs and we have shown that it interacts very well with tramadol. The following table shows that carrageenan is effective as a complexing agent with tramadol in retarding tramadol release.

Table 1 Complexation with lambda carrageenan to reduce the release duration gap ($T_{80}$ ratio)

| | Matrix | A (non-complex) | B (Lambda) | C (Kappa) | D (EC) |
|---|---|---|---|---|---|
| $T_{80}(h)$ | Acetaminophen | 17.7 | 20.0 | 17.6 | 18.8 |
| | Tramadol | 7.3 | 14.3 | 8.6 | 8.7 |
| $T_{80}$ ratio | | **2.4** | **1.4** | **2.0** | **2.2** |
| *n* | Acetaminophen | 0.658 | 0.708 | 0.524 | 0.672 |
| | Tramadol | 0.471 | 0.542 | 0.437 | 0.439 |

As shown in the above Table 1, lambda carrageenan had the lowest value $T_{80}$ ratio (1.4). $T_{80}$ means the time when the cumulative dissolution of APAP (and similarly for tramadol if the drug is tramadol) reaches 80%. $T_{80}$ ratio means ($T_{80}$ of APAP/ $T_{80}$ of tramadol). The lowest value of $T_{80}$ ratio (1.4) in the lambda carrageenan formulation means that the dissolution gap between two active pharmaceutical ingredients (APIs), i.e., drugs, was effectively reduced the most in the this formulation. For comparison, $T_{80}$ ratio was 2.0 for the formulation with Kappa carrageenan, 2.2 for the formulation with ethyl cellulose (EC), and 2.4 for non-complex formulation. Thus, ethyl cellulose can also act as a retarding agent, but it is less effective than carrageenan. The diffusion exponent n (described below) for lambda carrageenan also showed more zero order characteristics.

Other anionic materials that can be used for complexing with tramadol include alginic acid, carboxymethyl cellulose, etc. However, such other anionic materials have complexing forces that are weaker than carrageenan. Other sulfated or sulfonated polysaccharides or polymers, including dextran sulfate or strong cationic exchange resin (AMBERLITE IRP69) can be an anionic material for complexing with tramadol.

In forming the tramadol complex, the weight ratio of tramadol material to the anionic polymeric material (such as carrageenan) generally range from about 1: 0.1 to about 1: 100, preferably about 1: 0.5 to about 1:10.

In the compacted solid dosage form, the APAP and the tramadol material are generally present in a weight ratio of APAP to tramadol material from about 20:1 to 1: 1, preferably about 5: 1 to 10:1, even more preferably about 6:1 to 9:1. Further, in an immediate release (IR) layer, the APAP and the tramadol material are generally present in a weight ratio of APAP to tramadol material is about 20:1 to 1:1, preferably about 5:1 to 18:1, more preferably about 10:1 to 16:1. We have found that with APAP to tramadol ratios of such ranges we were able to provide coordinated delivery of the two drugs with very close wt% cumulative release rates in a single tablet, providing substantially more than 30 wt% cumulative release within the first hour of delivery, sustaining to about 12 hours of extended delivery.

The IR layer that can be used for attaching to the ER material can include APAP, tramadol, and excipients such as disintegrants, binders and fillers. Materials such as magnesium stearate, powdered cellulose, corn starch, gelatinized starch, sodium starch can be used. Easily soluble binders such as gelatinzed starch, polyvinylpyrrolidone, gum, etc., helps to temporarily hold the different ingredients together until the formulation enters an aqueous environment. Such binders will quickly solubilize and allow the IR layer to come apart, releasing the drugs. Disintegrants such as sodium starch glycolate, powdered cellulose, fibrous cellulose, and powdered silica helps the layer to fall apart readily and more uniformly as the binder is dissolved away. Lubricants such as magnesium stearate, sodium stearyl fumarate can also be used.

Regarding the ER layer, disregarding the IR layer next to it, we were able to achieve release that when the accumulative release of tramadol is 40 wt%, the accumulative release of APAP is less than 25wt% different from the accumulative release of tramadol. We were also able to achieve release that in the sustained release starting from when the accumulative release of tramadol is 40 wt%, the wt% accumulative release of APAP is never more than 20wt% different from the wt% accumulative release of tramadol. We were also able to achieve release that when the accumulative release (in wt%) of tramadol is 40 wt%, the accumulative release (in wt%) of APAP is never more than 10wt% different from the accumulative release (in wt%) of tramadol. We were further able to achieve that in the sustained release after the first hour for a sustained release of at least 12 hours, the wt% accumulative release of APAP is never more than 10wt% different from the wt% accumulative release of tramadol. The sustained release accumulative releases can be determined by United States Pharmacopeia Apparatus II (USP II) Paddle method at 37 C at 50 rpm/900ml in vitro in a buffer solution for dissolution at pH 6.8 (standard USP simulated intestinal fluid, but without enzyme).

The portion of a tablet (such as one of the layers of a bi-layer tablet) according to the present invention preferably is prepared by a compression process of particles, with the particles or granules containing the active pharmaceutical ingredient and other excipients that may be present. The materials of the extended release layer are compressed into a compacted unit before covering with an immediate release layer, such as that shown in FIG. 1A, etc. These particles preferably have an average particle diameter of about $30\mu$ to $3000\mu$, more preferably about $100\mu$ to $1000\mu$, and most preferably about $150\mu$ to $400\mu$. The term "particle diameter" generally refers to the larger dimension of a particle when the particle is not spherical in shape.

It is preferred that the tramadol complex has particle size with particle diameter of about 30μ to 3000μ, more preferably about 100μ to 900μ, and most preferably about 150μ to 300μ.

The extended release layer or core can contain various water-insoluble materials as excipients. Examples of such water insoluble materials include polymers which can be hydrophobic polymers. Examples of useful water-insoluble materials include, but are not limited to, one or more of ethyl cellulose, butyl cellulose, cellulose acetate, cellulose propionate, and the like.

The ER layer or core can be produced by combining the active pharmaceutical ingredient and at least one agent capable of restricting release of the active ingredient, and other ingredients. For example, the ER layer or core may contain a wide variety of excipients, including diluents, glidants, binders, granulating solvent, granulating agents, anti-aggregating agents, buffers, lubricants. For example, optional diluents can include, one or more of sugars such as sucrose, lactose, mannitol, glucose; starch; microcrystalline cellulose; sorbitol, maltodextrin, calcium salts and sodium salt, such as calcium phosphate; calcium sulfate; sodium sulfate or similar anhydrous sulfate; calcium lactate; other lactose material such as anhydrous lactose; and lactose monohydrate. One preferred diluent is lactose.

Binder(s) can be used to bind the materials (such as those in the ER material) together. Suitable binders can include one or more of the following exemplary materials, polyvinyl alcohol, polyacrylic acid, polymethacryic acid, polyvinyl pyrrolidone, sucrose, sorbitol, hydroxyethyl cellulose, hydroxypropylmethyl cellulose (HPMC), hydroxypropyl cellulose, polyethylene glycols, gum arabic, gelatin, agar, polyethylene oxide (PEO), etc. HPMC is preferably used in the formulation as it tends to aid in extending the release time. HPMC E5 has much lower MW than HPMC K4M and serves as a binder. The viscosity is about 5 cps in 2% solution for HPMC E5 and about 4000 cps for HPMC K4M. Due to the difference in viscosity, HPMC E5 is preferred as a binder for immediate release (IR) granulation and HPMC K4M is preferred for extended release formulation. Another preferred material is polyethylene oxide. In the drug release in a formulation, first, water penetrates into the polymer; then polymer chain relaxation takes place on response to water penetration. As a result, drug molecules diffuse through the polymer as the material swells. Binders like HPMC and PEO also have the property of forming a gel that hinders the penetration of liquid to the drug such that the release of drug from the formulation is retarded. Due to their high MW and viscosity, HPMC and PEO are useful to the extended release formulations.

Lubricants and anti-aggregating agents include, but are not limited to, one or more of talc, magnesium stearate, calcium stearate, colloidal silica, stearic acid, waxes, hydrogenated vegetable oil, polyethylene glycols, sodium benzoate, sodium laurylsulfate, magnesium laurylsulfate and dl-leucine. A useful lubricant is a silica material, e.g., AEROSIL, which is a commercially available colloidal silicon dioxide that is submicroscopic fumed silica with particle size of about 15 nm.

Optionally, one or more outer coatings may be applied over the tablet to provide protection during packaging, handling and aid in the swallowing process. Such outer coatings preferably disintegrate quickly to enable the immediate release layer to quickly release the active ingredients therein. The coating can include one or more tablet coating materials. Suitable coating materials include gelatin, saccharides (e.g., monosaccharides, disaccharides, polysaccharides such as starch, cellulose derivatives). Other useful coating materials include polyhedric alcohols such as xylitol, mannitol, sorbitol, polyalkylene glycols, and the like. Such coating materials and methods of their use are known to those skilled in the art. Examples of useful coating material are SURELEASE and OPADRY (both available from Colorcon, West Point, Pa., USA). The equipment and method of coating a tablet is well known in the art of tablet making. Further, optionally, waxy material such as Carnauba wax can be used as a surface finish to provide a shinier surface.

The process of producing the dosage form tablet of the present invention employs traditional techniques in forming a tablet. In one aspect, an extended release layer is formed of the extended release material and then covered with an immediate-release layer, and optionally, covered with one or more outer coatings. The ER material can also be a core of a tablet. The ER material can be formed by compressing the ingredient particles together into a compacted form. Preferably the compacted form of an embodiment of the invention has a hardness of about 4 to 20 KP/cm$^2$. Further, the particulate or granular forms of the ingredients can be formed by granulation in one or more processes of suitable techniques, which may include granulating in granulators of various kinds: a low shear granulator, fluidized bed granulator, high shear granulator, and the like.

Tablets of the present invention may be made by any means known in the art. Conventional methods for tablet production include direct compression ("dry blending"), dry granulation followed by compression, and wet granulation followed by drying and compression.

Preferably the tablet or a layer of the tablet is formed by the direct compression method, which involves directly compacting a blend of the active ingredient. For example, after blending, the powder blend is filled into a die cavity of a tablet.press (such as a rotary press), which presses the material into tablet form. As used herein, the tablet can have shape of a traditional elongate shape with rounded rectangular cross section, a spherical shape, a disk-pill shape, and the like. The materials are compressed into tablet shapes to a hardness of preferably between about 2 and 6 KP, with a preferred value being about 4 KP when the tablet is dry. In this invention, IR or ER layers or tablets were compressed through wet granulation method, and the hardness is 6 KP or more.

For the particles to be compressed, following production of the particles or granules, the materials can be dried under sufficient conditions to provide granules preferably having not more than 0.5 %wt water. In this invention, LOD (loss on drying) range of IR and ER granules results in moisture level of from 1.0% to 3.0% after drying. The materials can be dried at a preferred temperature of at about 50° C. Drying temperature range is about 40°C to 50°C preferably for suitable length of time, e.g., 12 - 16 hours to remove liquid, such as solvent and/or water. In lab scale, drying time is 12 - 16 hours. In industrial scale, drying time can be shorter, e.g., about 0.5 to 2 hours using fluid bed dryer.

In a bi-layer tablet, one layer can be deposited on the other layer, e.g., a layer of IR material can be deposited or attached on an ER layer or vice versa. Similarly, a dosage form with an ER layer sandwiched between two layers of IR material can be formed with the same method. Similarly, a surrounding layer of IR material can be deposited on a core to form an ER tablet with an IR layer surrounding the ER core so that the tablet can provide immediate release as well as sustained release for therapeutic relief to the patient.

Equipment and methods of forming of tablets with layers or tablets with a surrounding layer on a solid core in tablet manufacturing are well known in the art. For example, the immediate release layer on the core of extended release core can be achieved by a variety of granulation processes. Further, a bi-layer tablet can be made by using a bi-layer forming press. One way to form a bi-layer tablet is to compress granules or particles for one layer (e.g., the ER material) into a layer and then compress granules or particles for the other layer (e.g., the IR material) thereon to form a bi-layer tablet-like structure. To form a tri-layer tablet, the third layer (e.g., an IR layer) can be compressed on the selected side (e.g., the ER side) of the bi-layer tablet-like structure.

Generally, of the active ingredient in the whole tablet of the present invention, about 30 wt% to 90 wt%, preferably about 40 wt% to 80 wt%, more preferably about 50 wt% to 70 wt% of the APAP is in the ER core of the tablet. On the other hand, generally, about 30 wt% to 100 wt%, preferably about 50 wt% to 90 wt%, more preferably about 60 wt% to 80 wt% of the tramadol is in the ER core of the tablet. The balance of the active ingredients of APAP and tramadol can be in the IR layer next to the ER layer, to provide a quick rise of serum level of the drugs for therapeutic effect.

Procedures and Equipment

The following set forth typical, exemplary equipment and procedures that can be used to make, evaluate and use the dosage forms of the present invention. Lambda ($\lambda$) carrageenan is mentioned as illustrative example. Matrix tablets were prepared by wet granulation method. The detailed composition of various formulations is given in tables that will be presented below. In general, in the process of making the dosage form, tramadol HCl was dissolved in 60% ethanolic solution (1:1.5, w/v), and the complex was prepared by adding $\lambda$-carrageenan slowly to the resultant tramadol HCl solution with mixing in a wide-mouth vessel using a stirrer. Then, pre-blended APAP/HPMC powders were mixed with the complex to get a consistent wet paste. The paste was passed through a 1.0 mm-mesh screen, followed by drying at 45°C overnight. The dried granules were sieved through a 1.0 mm-mesh screen, and then blended with matrix forming polymers and other excipients including Mg stearate. Tablets of approximately 600 mg weight each were compressed from these granules using a rotary tablet press equipped with 19.5mm X 8.5mm oval punch and die set. The compression force was approximately 20KN and the hardness and thickness of tablets were approximately 7-10 KP and 3.9mm, respectively. All the preparations were stored in airtight containers at room temperature for further study.

K5SS mixer (Kitchen Aid, USA) was used for mixing and kneading the active ingredients and excipients. AR400 type FGS (Erweka, Germany) granulator was used for the granulating and sieving compounds. ZP198 rotary tablet press (Shanghai Tianhe Pharmaceutical Machinery Co., Ltd., China) was used for compressing the tablets, respectively. VK7000 (VANKEL, Germany) Dissolution System was used for in vitro dissolution testing of the compressed tablets, and LC-10A HPLC of SHIMADZU was used for quantitative analysis. Dissolution tester can be used for both USP I (basket) method and USP II (paddle) method. The description of USP methods of dissolution can be found in "Dissolution", The United States Pharmacopeia, 30th ed., pp. 277-284, The United States Pharmacopeial Convention, Rockville, MD (2007). It has been known in the art that dissolution tests such as USP I and USP II give reasonable prediction of dissolution of drugs in vivo in the gastrointestinal track of a human patient. FDA has added USP dissolution as one of the required tests for oral formulation development due to the *in-vitro/in-vivo* correlation successes. See, for example, (1) Dressman, Jennifer B.; Amidon, Gordon L.; Reppas, Christos; Shah, Vinod P, Abstract of "Dissolution testing as a prognostic tool for oral drug absorption: immediate release dosage forms", Pharmaceutical Research (1998), 15(1), 11-22, Plenum Publishing Corp.; (2) Shah, Vinod P., Abstract of "The role of dissolution testing in the regulation of pharmaceuticals: the FDA perspective", Pharmaceutical Dissolution Testing, (2005), 81-96, Taylor & Francis, Boca Raton , Florida; and (3) Uppoor, V. R. S., Abstract of "Regulatory perspectives on in vitro (dissolution)/in vivo (bioavailability) correlations", Office of Clinical Pharmacology and Biopharmaceutics, FDA, CDER, Rockville, MD, USA, Journal of Controlled Release (2001), 72(1-3), 127-132, Elsevier Science Ireland Ltd.

A typical carrageenan is $\lambda$-carrageenan. $\lambda$-carrageenans (VISCARIN® GP109, VISCARIN® GP209) were obtained from FMC BioPolymers. HPMC 2910 (METHOCEL® K4M,), HPMC 2208(METHOCEL™ E5, METHOCEL™ E15) and Polyethylene oxide (POLYOX® WSR N12K) were provided by COLORCON.

In vitro drug release studies from the prepared matrix tablets were conducted for a period of 12 hours using a VK7000 Dissolution System according to USP II (Paddle) method under condition of 50-100 rpm/900ml at 37±0.5 °C with dissolution media (pH 1.2, pH 4.0, pH 6.8 buffer solution and distilled water, prepared according to USP). The pH 6.8 buffer was the same composition as USP simulated intestinal fluid (SIF) without enzyme; and the pH 1.2 buffer was the same composition as USP simulated gastric fluid (SGF) without enzyme; the pH 4.0 buffer was made with 0.05mol/l acetic acid and 0.05mol/l sodium acetate and adjusted to pH 4.0. The dissolution media sample (pH 1.2, pH 4.0, pH 6.8 buffer solution and distilled water) was taken at regular intervals to be filtered by 0.45$\mu$m membrane and the concentrations of both tramadol HCl and APAP in the release medium were measured by an HPLC, the conditions of which are as follows. Xterra RP8 (4.6 X 5.0mm, 5$\mu$m, Waters, USA) was used as column for HPLC analysis, and 0.5% NaCl aqueous solution / methanol (85/15) solution was used as mobile phase. Flow rate of the mobile phase was 1ml/min and injection volume was 10 $\mu$l. SHIMADZU SPD-10A UV detector was used as detector and detection wavelength was set at 275nm.

The amounts of drug present in the samples were calculated using appropriate calibration curves constructed from reference standards. Drug dissolved at specific time period was plotted as percent release versus time curve. The dissolution data were fitted according to the following well-known exponential equation (Korsmeyer equation in mathematical modeling), which is used in the art to describe the drug release behavior from polymeric systems.

$$M_t/M_\infty = kt^n$$

where $M_t/M_\infty$ is the fractional drug release at time $t$; $k$ is a release rate constant incorporating the macromolecular polymeric systems and the drug, and the magnitude of the release exponent "$n$" is the diffusional exponent indicative of the drug release mechanism. The value of $n$ for a tablet, $n = 0.45$ indicates a classical Fickian (Case I, diffusion-controlled drug release), $0.45 < n < 0.89$ for non-Fickian (Anomalous, drug diffusion and polymer erosion release), $n = 0.89$ for Case II (Zero order, erosion - controlled release) and $n > 0.89$ for super case II type of release. The anomalous transport (Non-Fickian) refers to a combination of both diffusion and erosion controlled-drug release.

Model independent approaches (*i.e.*, dissolution efficiency (DE) and mean dissolution time (MDT) were also used to compare differences in drug release extent and rate among the prepared formulas, and translate the profile difference into a single value:

$$DE\ (\%) = 100 \times \frac{\text{Area Under the dissolution Curve}_{(dissolution,\ 0\text{-}12h)}}{(100\%\ \times\ 12h)}$$

which is defined as the area under the dissolution curve up to a certain time, t, expressed as a percentage of the area of rectangle described by 100% dissolution in the same time. MDT is a measure of the dissolution rate: the higher the MDT, the slower the release rate.

$$MDT = \frac{\sum_{i=1}^{i=n} t_{mid} \times DM}{\sum_{i=1}^{i=n} DM}$$

where $i$ is the dissolution sample number variable, n is the number of dissolution sample times, $t_{mid}$ is the time at the midpoint between sampling time $i$ and $i$-1, and $\Delta M$ is the amount of drug dissolved between i and $i$-1.

EXAMPLES

In the following examples, tramadol HCl, i.e., racemic Cis - (2-(dimethylaminomethyl)-1-(3-methoxyphenyl)-cyclohexan-1-ol, $C_{16}H_{25}NO_2$) HCl was used to form the complex. In testing of optical rotation on the tramadol HCl, there was no rotation in linear polarised light. However, since complexing is an interaction of the cationic property of tramadol with the carrageenan, which has sulfate groups, it is expected that other enantiomers of tramadol HCl can complex similarly with carrageenan.

Example 1 Preparation of a tramadol complex

First, one gram of tramadol HCl was dissolved in 2 ml of deionized water. The resulted drug solution had an acidic pH. Next, 0.8 g of $\lambda$-carrageenan (VISCARIN GP-109 from FMC) was added into the drug solution and triturated for about 5 minutes, using a set of mortar/pestle to form tramadol complex paste. The paste was dried at 40°C in an oven overnight. The dried complex was then milled, using a set of mortar/pestle and passed through a 40-mesh screen. The tramadol content of the complex was measured, using HPLC. The target weight ratio of tramadol to the carrageenan was 1.0/0.8.

Example 2 Preparation of a tramadol complex

The complex preparation procedure of Example 1 was repeated in this example, except the ratio of tramadol to the carrageenan was 1.01/1.0

Example 3 Preparation of a tramadol complex

The complex preparation procedure of Example 1 was repeated in this example, except the ratio of tramadol to the carrageenan was 1.01/1.25.

Example 4 Release of tramadol with and without complexing

First, the excipients listed in Table 2 were passed through a 40-mesh screen. Then, the tramadol complex prepared in Example 1 or free tramadol was dry-blended with those screened excipients, in accordance with the compositions shown in Table 2. An amount of 600 mg of each dry blended material was compressed to a tablet using 9/32 inch tooling under about 1 metric ton of compression pressure. The pressure of 1 metric ton corresponds to 57 Mpa.

Table 2 Compositions A and B (wt%)

| Component | A | B |
|---|---|---|
| Tramadol HCl | 112.5 | |
| APAP | 54.2 | 54.2 |
| HPMC K4M | 15.0 | 15.0 |
| MCC | 17.3 | 7.3 |
| Mg Stearate | 1.0 | 1.0 |
| Complex in Example 1 | | 22.5 |

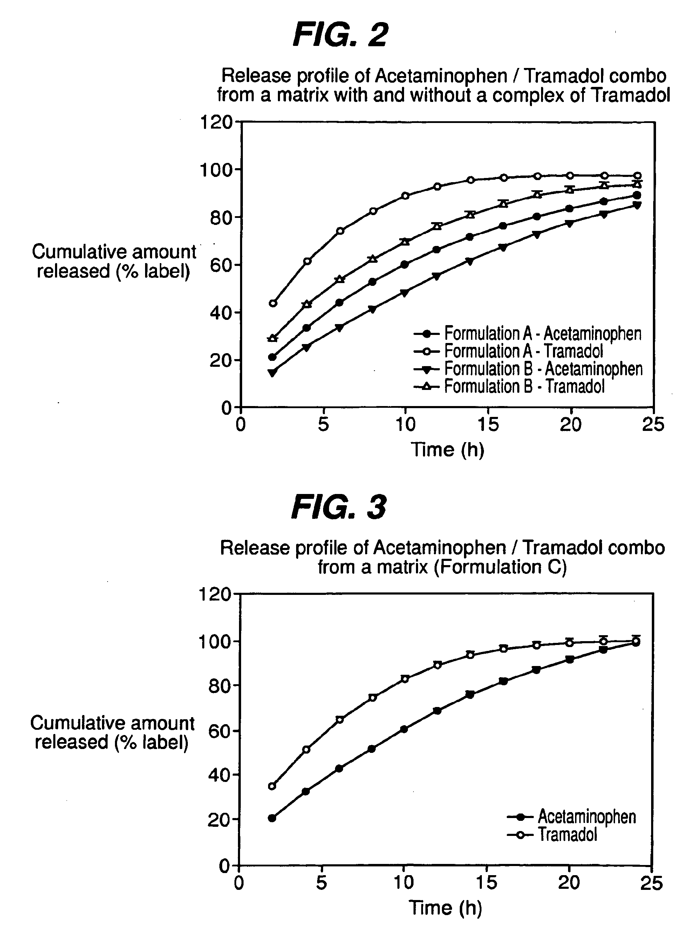

The release profiles of both tramadol and APAP were measured in a simulated intestinal fluid (standard pH 6.8 USP, without enzyme) at 50 rpm, using USP I method. The concentrations of tramadol and APAP in the release medium were measured using an HPLC method (Waters XTerra RP8, $5\mu m$, 4.6x50mm; with mobile phase of 85:15, v/v, 0.5%NaCl in water:MeOH). FIG. 2 shows the release profile of APAP/tramadol combination from a matrix in which the tramadol is and is not complexed. The curve with the black disks data points are the Formulation A APAP data, the open circles are the Formulation A tramadol data, the black triangles are the Formulation B APAP data, and the open triangles are the Formulation B tramadol data. The data show that the release rate of tramadol is much faster than that of APAP, with $T_{80}$, defined as the time for 80% of a drug released, being 7.3 and 17.7 hrs, respectively. There was a release duration gap between these two drugs, with the $T_{80}$ ratio being 2.4. For Formulation B, where tramadol was complexed with the carrageenan, the release duration gap was significantly reduced. The $T_{80}$ ratio was reduced from 2.4 to 1.4, with p-value of $< 0.0001$. Thus, complexing with carrageenan delays the release of tramadol. See Table 1 above.

Example 5 Effect of HPMC

The tablet preparation procedure and release methodology shown in Example 4 were repeated in this Example, except the tablet compositions were changed in order to provide a wide range of release durations. Table 3 shows the tablet compositions used.

Table 3 Compositions C, D, and E: varying HPMC K4M amount (wt%)

| Component | c | D | E |
|---|---|---|---|
| Complex in Example 1 | 22.5 | 22.5 | 22.5 |
| APAP | 54.2 | 54.2 | 54.2 |

(continued)

| Component | c | D | E |
|---|---|---|---|
| HPMC K4M | 10.0 | 5.0 | 0.0 |
| Lactose | 12.3 | 17.3 | 22.3 |
| Mg Stearate | 1.0 | 1.0 | 1.0 |

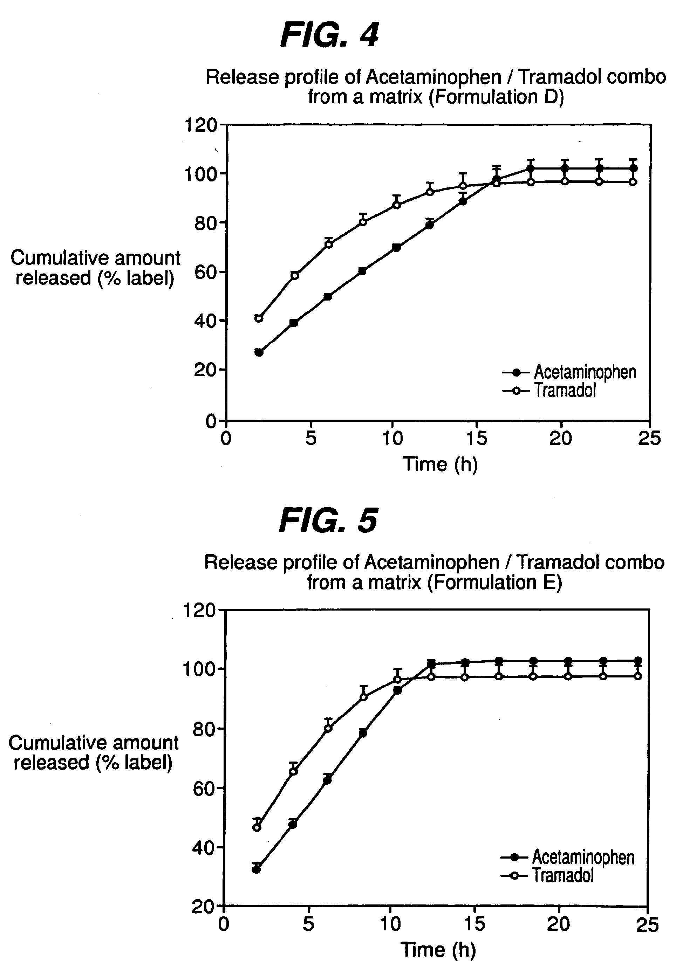

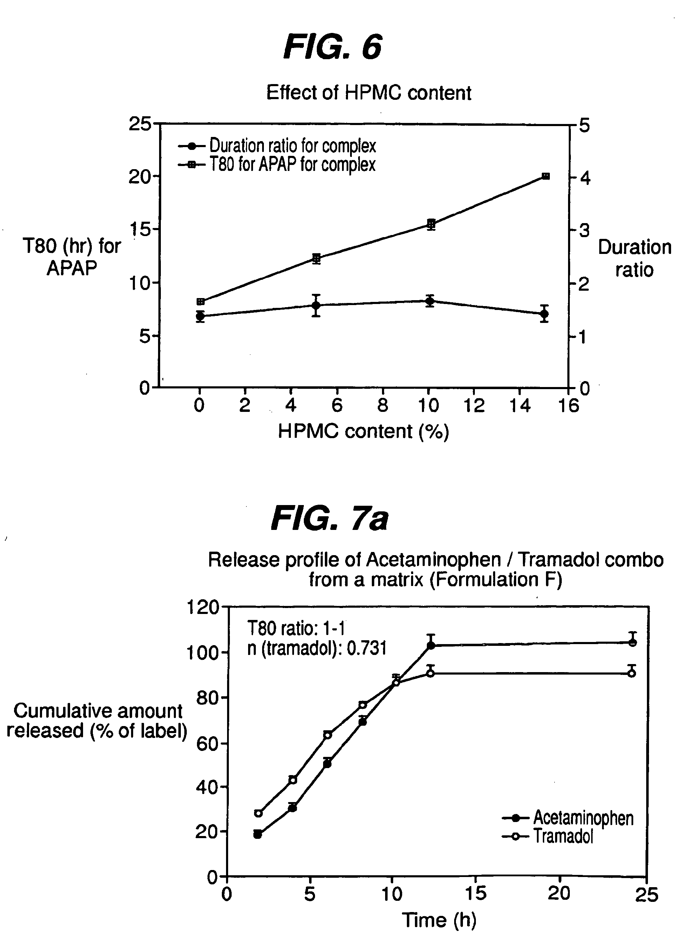

FIGs. 3, 4 and 5 show the release profiles for Formulations C, D and E, respectively, having different amount of hydroxypropylmethyl cellulose K4M (HPMC K4M). The black disks are the APAP data, and the open circles are the tramadol data. The $T_{80}$ for APAP and the duration ratio for the tramadol were plotted in FIG. 6. The black disks are the APAP data, and the open circles are the tramadol data. FIG. 6 shows that with the formulations containing tramadol complex, the HPMC content greatly influenced the APAP release duration (increasing the amount of HPMC increased $T_{80}$) but has no significant effect on the duration ratio.

Example 6 Effect of complexing tramadol

Table 4. Compositions F (without complexing) and G (with complexing) showing wt%

| Component | F | G |
|---|---|---|
| Tramadol HCl | | 12.5 |
| APAP | 54.2 | 54.2 |
| HPMC E5 | 5.0 | |
| HPMC K4M | | 10.0 |
| Lactose | 11.7 | 22. 3 |
| Mg Stearate | 1.0 | 1.0 |
| Complex in Example 3 | 28.1 | |

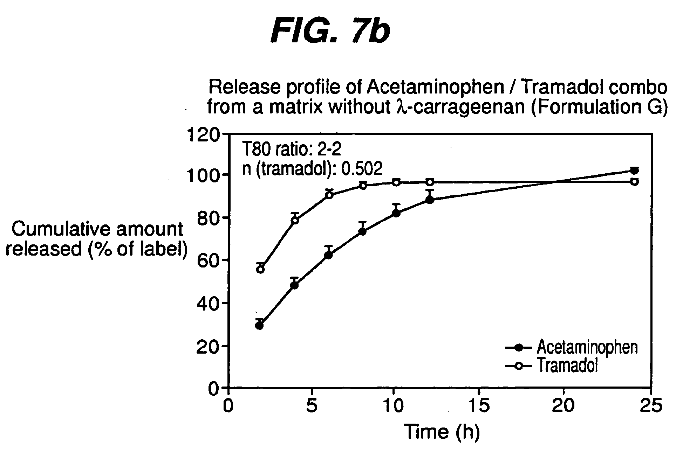

The preparation procedure was identical to that described in Example 4 above with the formulations F and G according to Table 4. FIGs. 7a and 7b show the release profiles of tramadol and APAP for composition F and G, respectively. The black disks are the APAP data, and the open circles are the tramadol data. These two formulations had similar $T_{80}$ profiles for APAP, but a big difference in tramadol release profiles. With complexation (Formulation F), the synchronized release of tramadol and APAP was achieved, with the release duration ratio being 1.1. In addition, the diffusional release exponent (n) for complexed tramadol was 0.731, compared to 0.502 for that without complexation. The increase in the value of n indicated that the tramadol release became closer to zero-order (constant rate) delivery.

Example 7 Immediate release material

The same tablet preparation procedure of Example 4 was repeated in this example, except that formulation was that of an immediate release (IR) material, according to Table 5. The ingredients were passed through a 40 mesh screen before dry blending to ensure homogenous mixing. We prepared an immediate released tablet in which 365.2 mg of the composition was compressed into an IR tablet, using 0.75x0.32 inch caplet tooling under about 1 metric ton of pressure (corresponding to 57 Mpa) by a CAVER compressor. Each IR tablet contained 325 mg of APAP. The tablet was shown to disintegrate rapidly, with more than 95% of APAP dissolved in a simulated gastric fluid (SGF) (i.e., standard pH1.5 USP without enzyme, dissolution done in the standard procedure with USP II method) in less than 15 minutes. Thus, it was shown that an IR material was formed that would quickly release the APAP. This material can be used as an IR layer that is attached to an extended release composition that includes APAP and a complexed tramadol, as shown in FIG. 1A, FIG 1B, and FIG. 1C. As an outer layer in a tablet, it should disintegrate and release the drugs similarly quickly. In this present experiment, the IR layer 22 included no tramadol, but APAP was the only active analgesic ingredient. However, since the IR material dissolved so quickly, there is no reason to think that including tramadol will extend the drug release time to any significant degree. An IR layer with APAP and tramadol should dissolve in minutes, as compared to the ER material, which released APAP and tramadol over many hours.

Table 5. Compositions for immediate release material

| Component | Wt% |
|---|---|
| APAP | 89.0 |
| HPMC E5 | 5.0 |
| Sodium starch glycolate (PRIMOJEL) | 5.0 |
| Mg Stearate | 1.0 |

Example 8 Effect of HPMC E5 on hardness

Formulations F-No. 01A - 03A were prepared by using various HPMC E5 proportions as per formula given in Table 6A, to show the effect of HPMC E5 on tablet compressibility. Tablet hardness was higher with higher quantity of HPMC E5. The incorporation of 10 mg of HPMC E5 into ER layer granule was thus useful in producing a tablet of appropriate hardness, e.g., from about 6 to 12 KP.

Table 6A. Composition for the effect of HPMC E5 on compressibility.

| Ingredient (mg) | F-No. 01A | F-No. 02A | F-No. 03A |
|---|---|---|---|
| Tramadol HCl | 56.25 | 56.25 | 56.25 |
| APAP | 390 | 390 | 390 |
| λ-C (GP-109) | 70.4 | 70.4 | 70.4 |
| HPMC E5 | 0 | 5 | 10 |
| Mg stearate | 5.2 | 5.3 | 5.3 |
| Total | 521.85 | 526.95 | 531.95 |
| | | | |
| Hardness (KP) | 2-5 | 6-9 | 7-11 |

Example 9 Further examples of complex matrix - tablets

Matrix tablets were prepared by wet granulation method. The detailed composition of various formulations is given in Table 6B and Table 6C. Tramadol HCl was dissolved in 60% ethanolic solution (1:1.5, w/v), and the complex was prepared by adding lambda carrageenan slowly to the resultant tramadol HCl solution with mixing in a wide-mouth vessel using a stirrer. Then, pre-blended APAP/HPMC powders were mixed with the complex to get a consistent wet paste. The paste was passed through a 1.0 mm-mesh screen, followed by drying at 45°C overnight. The dried granules were sieved through a 1.0 mm-mesh screen, and then blended with matrix forming polymers and other excipients including magnesium (Mg) stearate. Tablets of approximately 600 mg weight each were compressed from these granules using a rotary tablet press equipped with 19.5mm X 8.5mm oval punch and die set. The compression force was approximately 20KN and the hardness and thickness of tablets were approximately 7-10 KP and 3.9mm, respectively. All the preparations were stored in airtight containers at room temperature for further study. The tablet making method can also be adapted for making tablets with ingredients in the following examples by including the correct excipients.

Table 6B Compositions of Formulations F. No. 1 to F. No. 5

| Ingredients (mg) | F-No. 1 | F-No.2 | F-No.3 | F-No.4 | F-No.5 |
|---|---|---|---|---|---|
| APAP | 390 | 390 | 390 | 390 | 390 |
| Tramadol HCl | 56.25 | 56.25 | 56.25 | 56.25 | 56.25 |
| λ-C (GP-109) | 70.4 | 70.4 | 70.4 | 70.4 | 70.4 |
| λ-C (GP-209) | 0 | 0 | 0 | 0 | 0 |
| HPMC E5 | 10 | 0 | 0 | 10 | 10 |

(continued)

| Ingredients (mg) | F-No. 1 | F-No.2 | F-No.3 | F-No.4 | F-No.5 |
|---|---|---|---|---|---|
| HPMC E15 | | 0 | 0 | 0 | 0 |
| POLYOX WSR N12K | 0 | 0 | 0 | 67.35 | 33.675 |
| HPMC K4M | 67.35 | 50 | 50 | 0 | 33.675 |
| Lactose | 0 | 27.35 | 0 | 0 | 0 |
| AEROSIL 200 | 0 | 0 | 27.35 | 0 | 0 |
| Mg Stearate | 6 | 6 | 6 | 6 | 6 |
| Total weight | 600 | 600 | 600 | 600 | 600 |

Table 6C Compositions of formulations F. No. 6 to F. No. 12

| Ingredien ts (mg) | F-No.6 | F-No 7 | F-No. 8 | F-No 9 | F-No 10 | F-No. 11 | F-No. 12 |
|---|---|---|---|---|---|---|---|
| APAP | 390 | 390 | 390 | 390 | 390 | 390 | 390 |
| Tramadol HCl | 56.25 | 56.2 5 | 56.2 5 | 56.25 | 56.25 | 56.25 | 56.25 |
| $\lambda$-C (GP-109) | 70.4 | 71 | | 71 | 71 | 71 | 71 |
| $\lambda$-C (GP-209) | 0 | 0 | 71 | 0 | 0 | 0 | 0 |
| HPMC E5 | 10 | 0 | 0 | 0 | 0 | 0 | 0 |
| HPMC E15 | 0 | 10 | 10 | 10 | 10 | 10 | 10 |
| POLYOX WSR N12K | 30 | 30 | 30 | 30 | 30 | 20 | 50 |
| HPMC K4M | 30 | 30 | 30 | 25 | 20 | 20 | 20 |
| Lactose | 7.5 | 6.75 | 6.75 | 6.75 | 6.75 | 6.75 | 6.75 |
| AEROSIL 200 | 0 | 0 | 0 | 0 | 0 | 0 | 0 |
| Mg Stearate | 6 | 6 | 6 | 6 | 6 | 6 | 6 |
| Total weight | 600 | 600 | 600 | 595 | 590 | 580 | 610 |

Example 10 Retarding excipients

Hydrophilic polymers such as polyethylene oxide (PEO) and hydroxypropyl methylcellulose (HPMC) can be used as excipients for modifying release tablet formulations. The tablets can be made with the method of the above Example 9, which will be understood by one skilled in the art. Once in contact with a liquid, these polymers would hydrate and swell, forming a hydrogel layer that regulates further penetration of the liquid into tablet matrix and dissolution of the drug from within. Drug release from such a polymeric matrix is therefore achieved by diffusion, erosion, or a combination of both. Matrix tablets of ER layer were formulated at various contents of HPMC and PEO with the $\lambda$-carrageenan/tramadol HCl complex to achieve the release duration of approximately 10-12 hrs for BID dosing, see Table 7 (which includes Table 7A for APAP and Table 7B for tramadol HCl). The PEO used was POLYOX WSR N12K obtained from DOW chemical company. Its molecular weight (MW) is approximately 1,000,000 and viscosity range is 400-800 cps at 2% solution at 25°C for POLYOX WSR N-12K-NF. Table 7 lists dissolution parameters of each matrix tablet formulation obtained from various empirical equations. As observed from the table, the value of correlation coefficient ($R^2$) for all the formulations were high enough (>0.97) to evaluate the drug dissolution behavior by Korsmeyer model, and the values of "$n$" and k were found to vary with type and concentration of polymer. The value of release exponent "$n$" determined from the various matrices ranged from 0.43 to 0.88 for APAP and from 0.46 to 0.66 for tramadol HCl, indicating combined effect of diffusion and erosion mechanisms. When HPMC K4M alone was employed as a retarding agent in F-No. 1, tablet hardness was relatively low (less than 3 KP), which made compression difficult. However, the incorporation of lactose or AEROSIL 200 into a preparation as tablet fillers enabled the supplement of appropriate tabletting properties (F-No. 2 & 3).

Table 7A *In vitro* drug release and dissolution parameters of APAP

| | Diffusion exponent (*n*) | Release rate constant (*k*) | Correlation coefficient (R2) | MDT (h) | DE% |
|---|---|---|---|---|---|
| F-No.2 | 0.4301 | 0.2738 | 0.9744 | 4.57 | 57.57 |
| F-No.3 | 0.6156 | 0.1336 | 0.9912 | 5.05 | 39.41 |
| F-No.4 | 0.7933 | 0.1290 | 0.9946 | 5.25 | 51.85 |
| F-No.5 | 0.8655 | 0.0848 | 0.9990 | 5.84 | 39.34 |
| F-No.6 | 0.7739 | 0.1170 | 0.9967 | 5.19 | 46.29 |
| F-No. 7 | 0.7549 | 0.1255 | 0.9962 | 5.40 | 47.92 |
| F-No. 7 (75rpm) | 0.6146 | 0.1994 | 0.9874 | 4.82 | 59.28 |
| F-No. 7 (100rpm) | 0.6349 | 0.2080 | 0.9861 | 4.33 | 64.41 |
| F-No. 8 | 0.7501 | 0.1342 | 0.9966 | 5.28 | 49.96 |
| F-No. 9 | 0.8840 | 0.0972 | 0.9972 | 5.31 | 46.21 |
| F-No. 10 | 0.7075 | 0.1513 | 0.9968 | 5.14 | 52.57 |
| F-No. 10 (pH 1.2) | 0.7847 | 0.1877 | 0.9907 | 3.29 | 69.04 |
| F-No. 10 (pH 4.0) | 0.6884 | 0.1860 | 0.9890 | 4.43 | 63.62 |
| F-No. 10 (DW) | 0.7856 | 0.1755 | 0.9919 | 3.97 | 68.46 |
| F-No. 11 | 0.6014 | 0.1994 | 0.9880 | 4.92 | 56.94 |
| F-No. 12 | 0.6418 | 0.1534 | 0.9932 | 5.12 | 46.87 |

Table 7B *In vitro* drug release and dissolution parameters of tramadol HCl

| | Diffusion exponent (*n*) | Release rate constant (*k*) | Correlation coefficient (R2) | MDT (h) | DE% |
|---|---|---|---|---|---|
| F-No.2 | 0.4662 | 0.2869 | 0.9993 | 3.59 | 61.86 |
| F-No.3 | 0.5823 | 0.2247 | 0.9983 | 4.33 | 60.04 |
| F-No.4 | 0.5986 | 0.2133 | 0.9966 | 4.18 | 59.65 |
| F-No.5 | 0.6325 | 0.1822 | 0.9909 | 4.90 | 54.59 |
| F-No.6 | 0.5694 | 0.2075 | 0.9977 | 4.33 | 54.91 |
| F-No.7 | 0.6076 | 0.2171 | 0.9995 | 4.28 | 60.87 |
| F-No.7 (75rpm) | 0.5070 | 0.2757 | 0.9974 | 3.43 | 64.18 |
| F-No.7 (100rpm) | 0.5123 | 0.2909 | 0.9952 | 3.21 | 68.24 |
| F-No.8 | 0.5814 | 0.2217 | 0.9965 | 4.16 | 59.63 |
| F-No.9 | 0.6601 | 0.1964 | 0.9957 | 4.22 | 60.47 |
| F-No.10 | 0.5677 | 0.2415 | 0.9984 | 3.99 | 63.09 |
| F-No.10 (pH 1.2) | 0.6394 | 0.2919 | 0.9949 | 2.50 | 77.70 |
| F-No. 10 (pH 4.0) | 0.5822 | 0.2793 | 0.9944 | 3.39 | 74.35 |
| F-No. 10 (DW) | 0.6548 | 0.2474 | 0.9816 | 3.31 | 74.38 |
| F-No. 11 | 0.4969 | 0.2817 | 0.9986 | 3.58 | 64.51 |
| F-No. 12 | 0.5283 | 0.2321 | 0.9994 | 4.03 | 56.30 |

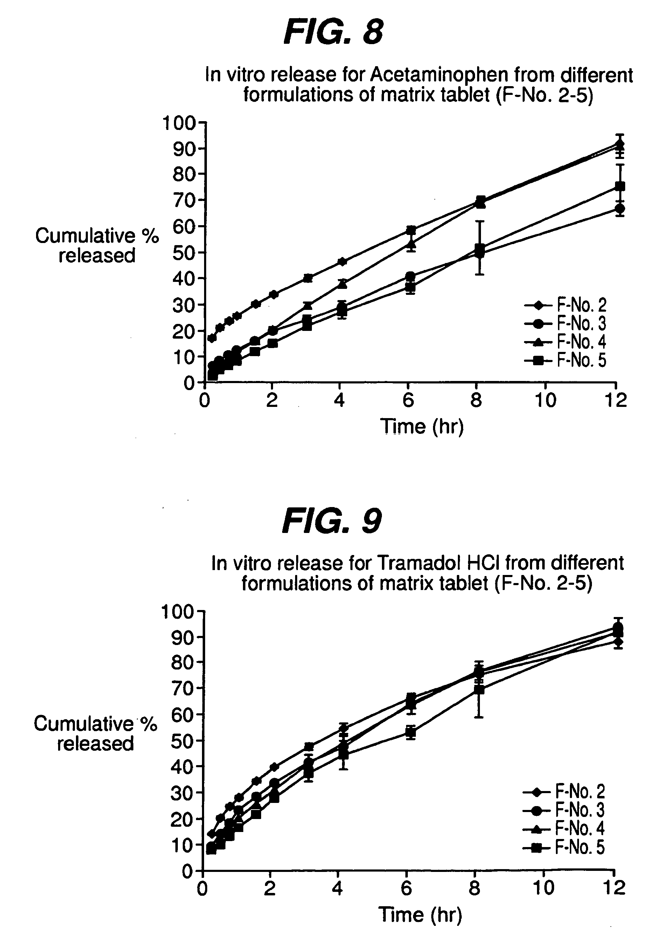

When HPMC K4M alone was employed as a retarding agent in F-No.1, tablet hardness was relatively low, which made compression difficult. However, the incorporation of lactose or AEROSIL 200 into a preparation as tablet fillers enabled the supplement of appropriate tabletting properties (F-No. 2 & 3). The use of PEO alone and the combined use of HPMC K4M and PEO as a retarding agent were also tested (F-No. 4 & 5). The dissolution was done at 50 rpm in a pH 6.8 buffer solution (simulated intestinal fluid, without enzyme). Dissolution percentage as a function of time for F-No. 2 - 5 are shown in FIG. 8 and FIG. 9.

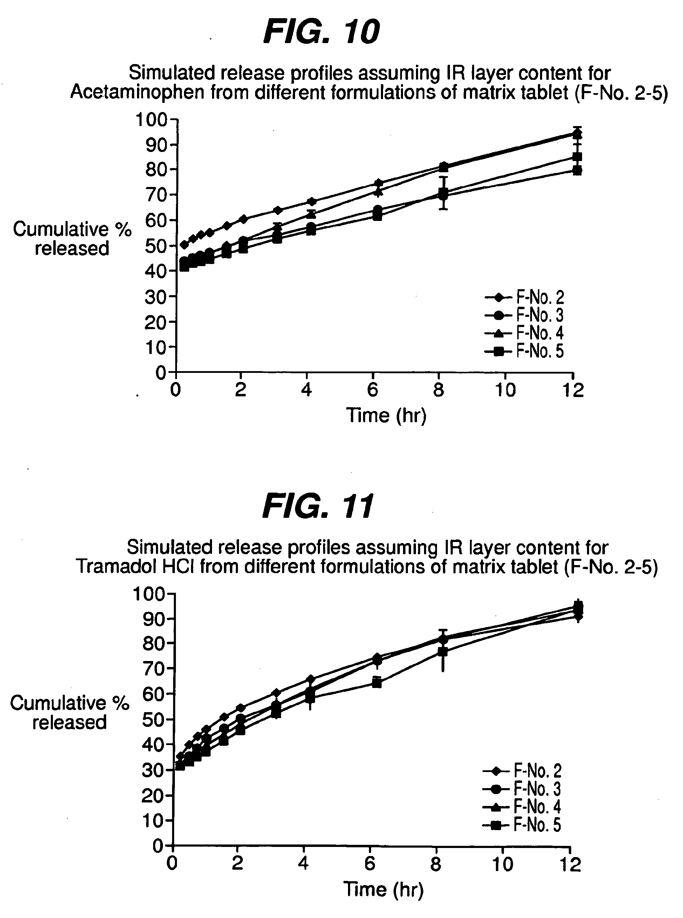

Simulated release profiles assuming IR layer content for (a) APAP and (b) tramadol HCl from different formulations of matrix tablet (F-No. 2 - 5) at 50 rpm in pH 6.8 buffer solution are shown in FIG. 10 and FIG. 11 (compared with FIG. 8 and FIG. 9 which are data without the assumption of having an IR layer). Data are represented as mean $\pm$ SD (n=3). The diamond data points represent the F-No.2 data. The circular data points represent the F-No.3 data. The triangular data points represent the F-No. 4 data. The square data points represent the F-No. 5 data. As shown in Example 4 above, an IR material can be formed that would release APAP quickly to bring up the amount of active ingredient released quickly. Similarly, we have also form IR materials that release APAP and tramadol quickly. We have demonstrated that if a layer of an IR material is used to form a bi-layer with a layer of ER material, the APAP and tramadol release can be approximated by assuming that the time it takes for APAP and the tramadol to be released is negligible. Structures of FIG. 1B and 1C should similarly release the drugs from the IR layer quickly. FIG. 10 and FIG. 11 show the simulated release profiles assuming that the tablet has an ER layer of compositions of those of FIG. 8 and FIG. 9 and an IR layer associated with the ER material, either as an outer layer or as one layer of a bi-layer structure. The cumulative % release is the release calculated as a percentage of the total amount of APAP (and tramadol) in the whole (e.g., bi-layer) tablet. FIG. 10 and FIG. 11 show that the cumulative % release of APAP was very close to that of tramadol from the IR/ER (e.g., bi-layer) tablet for a formulation. Thus, coordinated extended release of APAP and tramadol HCl could be obtained by the complexation.

The results also showed the combined use (F-No. 5) of PEO and HPMC K4M as a retarding agent showed the least DE% and greatest MDT among the above described matrices, indicating a higher drug retarding ability.

Use of PEO

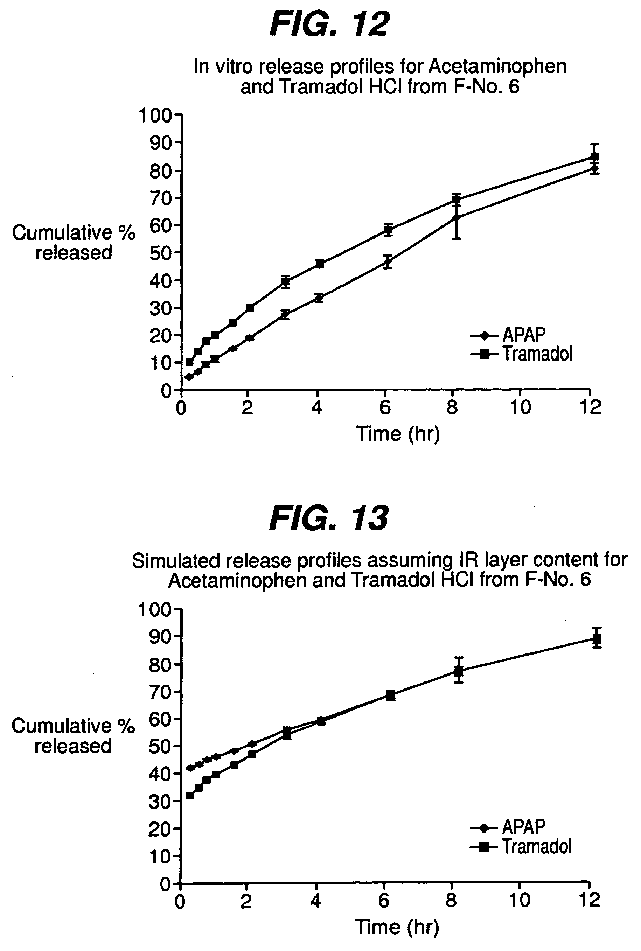

Formulations F-No. 5 and F-No. 6 showed the advantage of using HPMC K4M and PEO in obtaining small DE% and larger MDT. (F-No. 6) containing HPMC K4M and PEO at the ratio of 1:1. FIG. 12 shows the comparison of the cumulative release profiles of APAP and tramadol HCl. FIG. 13 shows the simulated release profiles of a bi-layer tablet with an IR outer layer and an ER core of F-No.6 calculated from the data of FIG. 12. In FIG. 12 and FIG. 13, the diamond data points represent the APAP data. The square data points represent the tramadol data. The release of APAP and tramadol HCl in FIG. 12 apparently follows Korsmeyer model (correlation $R^2$ = 0.9967 and 0.9977, respectively). From the release exponent ($n$ = 0.7739 and 0.5694 for APAP and tramadol HCl, respectively), the release mechanism seems to be an anomalous transport (Non-Fickian). The data show a substantially constant release rate adequate for an extended release. The extended release dosage form, enabling the constant release rate, likely reflects the summation of both drug diffusion and polymer erosion. Since both swelling and erosion occurred simultaneously in the matrix after placement in the dissolution media, substantially constant release resulted. Constant release in such situations occurs because the increase in diffusion path length due to swelling is compensated by continuous erosion of the matrix.

Different grades of $\lambda$-carrageenan

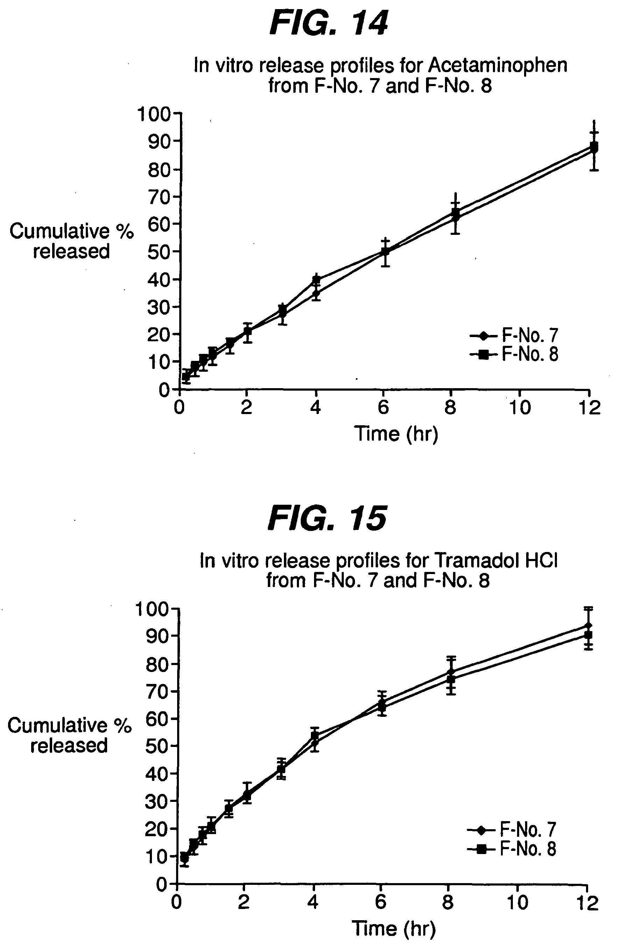

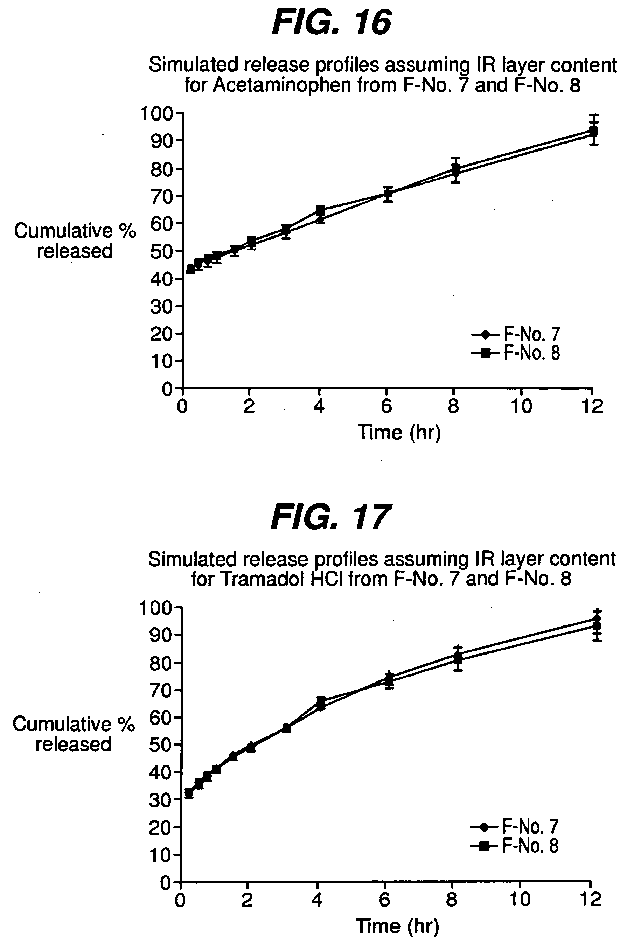

FIG. 14 shows a plot of cumulative amount of APAP released and FIG. 15 shows a plot of cumulative amount of tramadol HCl against time for the extended release formulations F-No. 7 and F-No. 8, which had different grades of $\lambda$-carrageenan. The diamond data points represent the F-No. 7 data. The square data points represent the F-No. 8 data. No significant difference was observed in drug release rate between matrices containing different grade of $\lambda$-carrageenan (VISCARIN® GP-109 and VISCARIN® GP-209), indicating that there is little difference in their complexation ability with tramadol HCl. FIG. 16 and FIG. 17 show the cumulative drug release of a simulated bi-layer tablet calculated based on the data of FIG. 15 and FIG. 15 respectively. Again, the release profile for the tramadol HCl was very close to that of the APAP, showing that the bi-layer dosage form with an extended release core of formulations F-No. 7 and F-No. 8 can produce coordinated extended release of the two drugs.

Effect of HPMC K4M

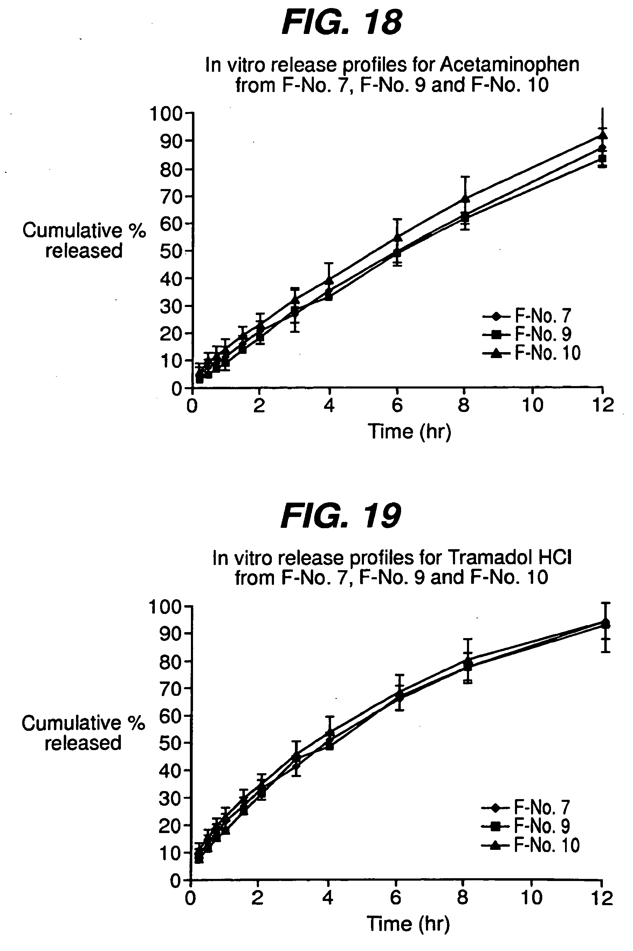

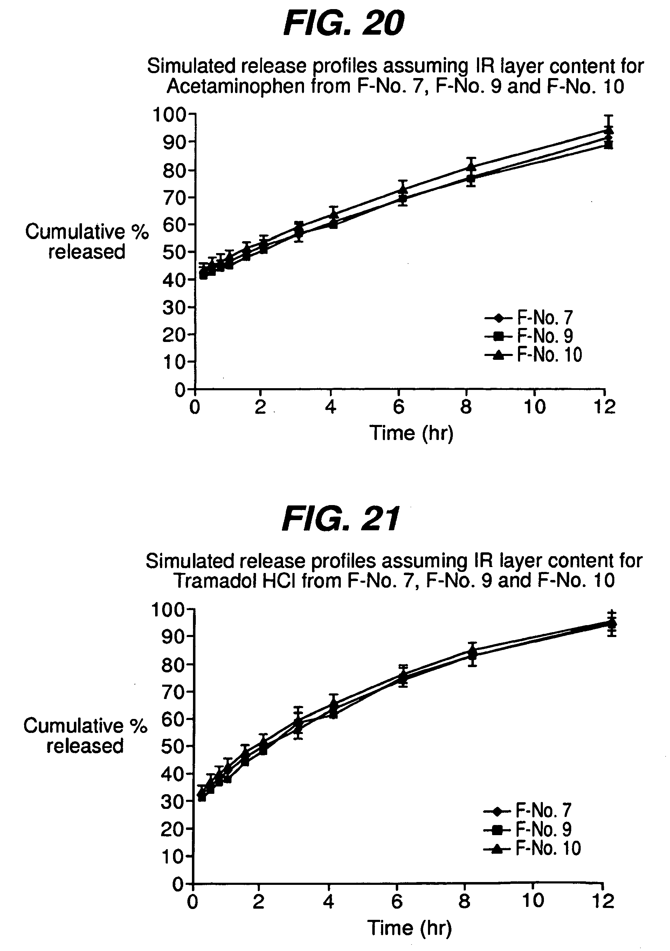

F-No. 7, F-no. 9 and F-No. 10 were formulated as an ER material by varying HPMC K4M proportions at the fixed amount of PEO (30 mg), to study the effect of retarding agent on drug release profile. All formulations showed a release over 10-12 h. FIG. 18 shows a plot of cumulative amount of APAP released and FIG. 19 shows a plot of cumulative amount of tramadol HCl against time for the extended release formulations F-No. 7, F-no. 9 and F-No. 10. FIG. 20 and FIG. 21 show the cumulative drug release of a simulated bi-layer tablet calculated based on the data of FIG. 18 and FIG. 19 respectively. The diamond data points represent the F-No. 7 data. The square data points represent the F-No. 9 data. The triangular data points represent the F-No. 10 data. The results show that increase amount of HPMC K4M retards the release of the drugs a little. Again, the release profile for the tramadol HCl was very close to that of the APAP, showing that the bi-layer dosage form with an extended release core of formulations F-No. 7, F-No. 9, and F-No. 10 can produce coordinated extended release of the two drugs.

Effect of PEO

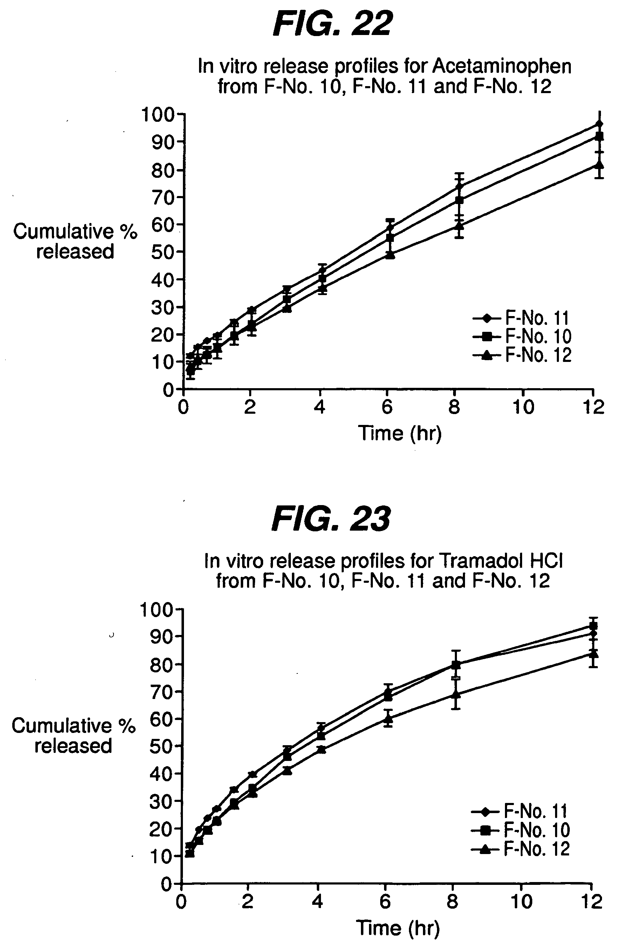

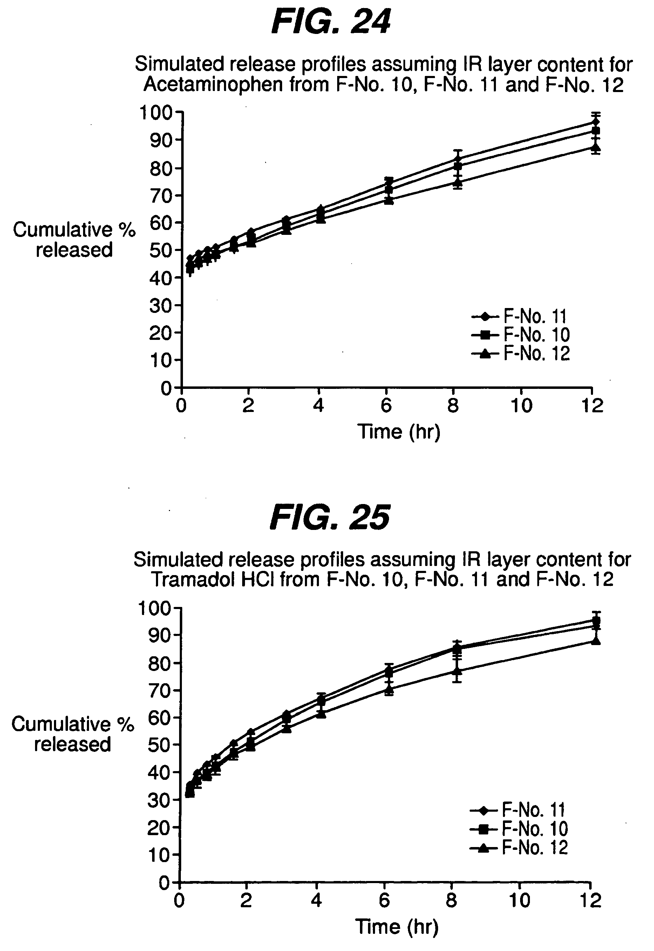

F-No. 10, F-no. 11 and F-No. 12 were formulated as an ER material by varying PEO proportions at the fixed amount of HPMC K4M (20 mg), to study the effect of retarding agent on drug release profile. All formulations showed a release over 10-12 h. FIG. 22 shows a plot of cumulative amount of APAP released and FIG. 23 shows a plot of cumulative amount of tramadol HCl against time for the extended release formulations F-No. 10, F-no. 11 and F-No. 12. FIG. 24 and FIG. 25 show the cumulative drug release of a simulated bi-layer tablet calculated based on the data of FIG. 22 and FIG. 23 respectively. The diamond data points represent the F-No. 11 data. The square data points represent the F-No. 10 data. The triangular data points represent the F-No. 12 data. The results show that increasing the amount of PEO retards the release of the drugs a little. Again, the cumulative release profile for the tramadol HCl was very close to that of the APAP, showing that the bi-layer dosage form with an extended release core of formulations 10, F-no. 11 and F-No. 12 can produce coordinated extended release of the two drugs.

Effect of pH

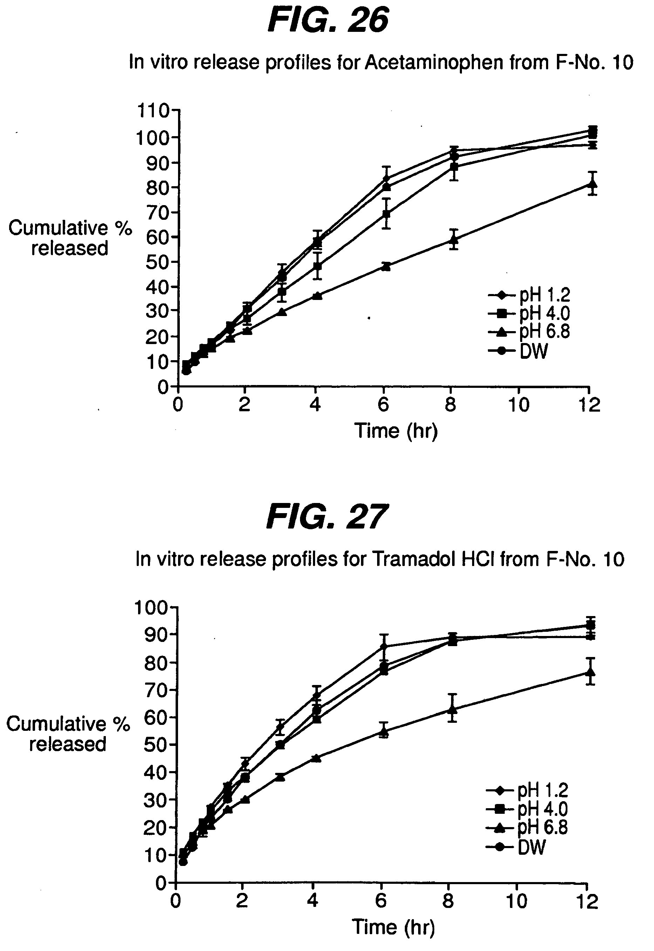

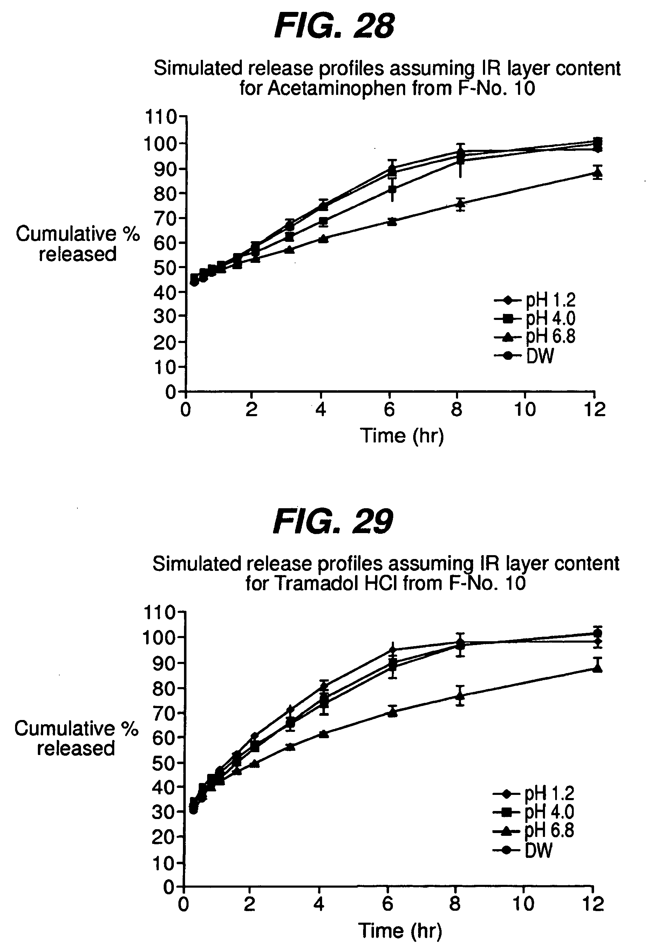

To study the effect of pH in the dissolution fluid on that the release rate of drugs from hydrophilic matrices, the dissolution rate was investigated with buffers at pH 1.2, pH 4.0, pH 6.8 and with distilled water for Formulation F-No.10 at 50 rpm. The data are shown in FIG. 26 and 27 for APAP and tramadol HCl respectively. The diamond data points represent the pH 1.2 data. The square data points represent the 4.0 data. The triangular data points represent the pH 6.8 data. The circular data points represent the distilled water (DW) data. For the formulation F-No.10, the release rates of both APAP and tramadol HCl were faster at acidic pH, in agreement that its value of MDT is lower and that of DE% higher in acidic condition. The results may be attributed to surface erosion or disaggregation of matrix tablet prior to gel layer formation around a tablet core in acidic media, resulting in faster release of drug. The pH 6.8 profiles were slower than the other ones. The release during the first hour was low for all the ER samples, indicating that such formulations would release only a small portion of the drugs when the tablets pass through the stomach. FIG. 28 and FIG. 29 show the cumulative drug release of a simulated bi-layer tablet in buffers of different pH and distilled water calculated based on the data of FIG. 26 and FIG. 27 respectively. Again, the results show that dosage form of coordinated release of APAP and tramadol can be formulated.

Effect of speed (rpm) of stirring

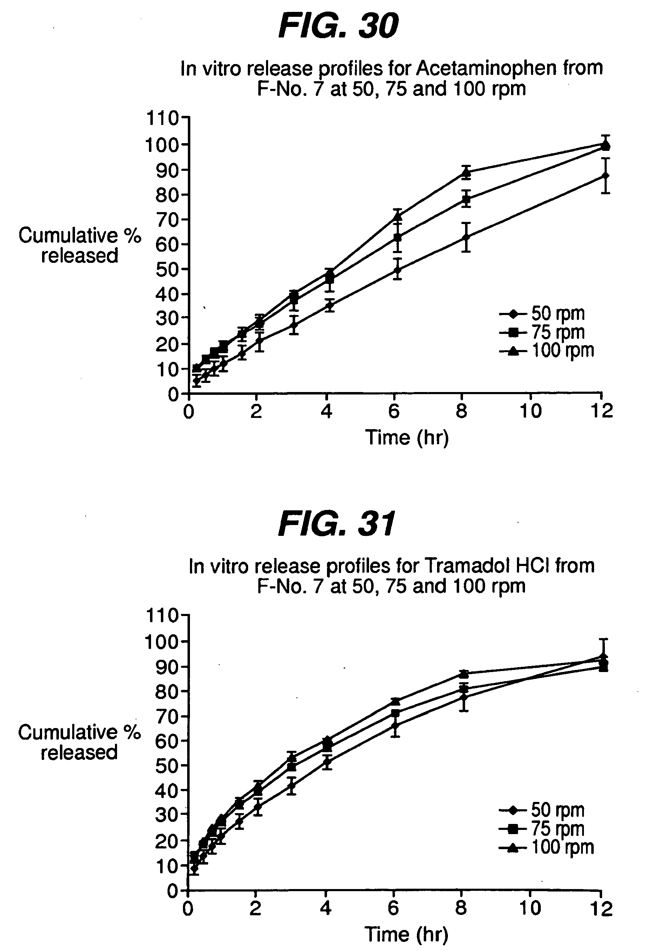

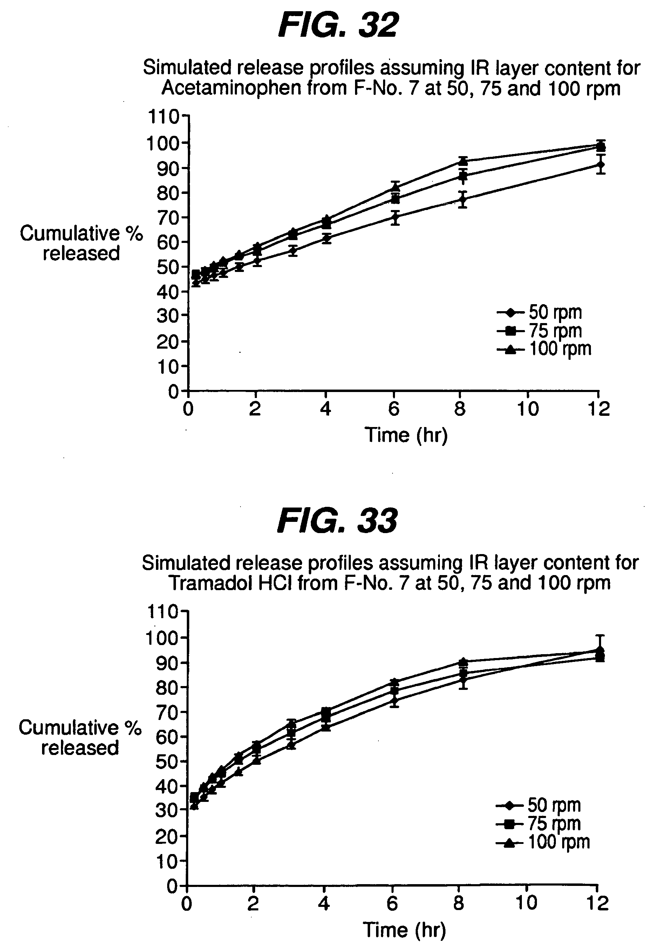

An exemplary ER material made of the Formulation F-No.7 was studied in dissolution runs at 50 rpm, 75 rpm and 100 rpm stirring speed. The data are shown in FIG. 30 and 31 for APAP and tramadol HCl respectively. The diamond data points represent the 50 rpm data. The square data points represent the 75 rpm data. The triangular data points represent the 100 rpm data. The overall rate of drug release from matrices is significantly higher at higher rpm, which is confirmed by smaller MDT, (4.33 h for APAP and 3.21 h for tramadol HCl) and higher DE% (64.41% for APAP and 68.24% for tramadol HCl) at 100 rpm for F-No. 7 than those at 50 rpm, which had MDT of 5.40 h for APAP and 4.28 h for tramadol HCl and DE% of 47.92% for APAP and 60.87% for tramadol HCl. Generally, hydrophilic polymer produces a hydrogel layer upon in contact with liquid; drug dissolution observes a combination of diffusion and erosion, with predominant in drug diffusion. However, higher rpm would result in more matrix erosion than polymer hydration, subsequently facilitating more drug diffusion and dissolution. FIG. 32 and FIG. 33 show the cumulative drug release of a simulated bi-layer tablet calculated based on the data of FIG. 30 and FIG. 31 respectively. Again, the results show that dosage form of coordinated release of APAP and tramadol can be formulated.

*In vitro* extended release of bi-layer tablet

Based on the results above, a bi-layer tablet having an IR layer with a layer of compacted tramadol HCl complex and APAP was made according to the composition of Formulation F-No.13 shown in Table 8, by adapting with the method of Example 9 to form the ER layer and depositing the IR layer thereon. This compression was done by using a double layer compress to compress the IR layer and the ER layer together as IR compression material and ER compression material were fed to a double layer compress simultaneously. Many presses for compressing material to form bi-layer or multilayer tablets are known and commonly used for making tablets. Typical presses, e.g., Carver press, can be used by those skilled in the art for making bi-layer tablets of this invention. Tablets of Formulation F-No. 13 were made in a pilot plant 38kg lot. Table 8 also shows the composition of an IR layer that is next to the layer of ER material. As shown in Table 8, an optional coating was also provided on the core tablet having IR and ER layers.

Table 8

| Ingredients (mg) | F-No. 13 |
|---|---|
| **IR layer** | |
| APAP | 260 |

(continued)

| Ingredients (mg) | F-No. 13 |
|---|---|
| **IR layer** | |
| Tramadol HCl | 17 |
| Powdered cellulose | 20.30 |
| Pregelatinized starch | 5.05 |
| Sodium Starch glycolate | 5.05 |
| Corn starch | 20.30 |
| Mg Stearate | 1.65 |
| **Sum of IR layer** | 329.35 |
| **ER layer** | |
| APAP | 390 |
| Tramadol HCl | 58 |
| λ-C (GP-109) | 72.5 |
| HPMC E15 | 10 |
| POPYOX WSRN12K | 30 |
| HPMC K4M | 30 |
| Mg Stearate | 5.96 |
| **Sum of ER layer** | 596.46 |
| **Coated layer** | |
| OPADRY | 25 |
| Carnauba wax | 0.04 |
| **Sum of Coated layer** | 25.04 |
| **Total Tab.weight** | 950.85 |

Table 9 shows the actual manufacturing data for making three pilot plant lots of bi-layer tablets of formula F-No. 13. The formula for three pilot plant manufactured batches produced tablets that met the acceptance criteria and exemplified a rugged and robust product. Water and/or ethanol were added as the other ingredients were being mixed for the corresponding layers as indicated in the table. The mixed materials were then compressed to form the corresponding layers. The water and ethanol were removed in a drying process for drying the tablets. These tablets also matched the performance of tablets that were evaluated at the lab scale and in the formulation development stage.

Table 9. Actual amount for three manufacturing batches

| Ingredient | Unit Formula (mg/Tab.) | Lot Quantity | Actual Lot Quantity | | |
|---|---|---|---|---|---|
| | | | Lot No. 001 | Lot No. 002 | Lot No. 003 |
| **Immediate Release layer** | | | | | |
| APAP | 260.0 | 31 kg 200 g | 31 kg 200 g | 31 kg 200 g | 31 kg 200 g |
| Tramadol HCl | 17.0 | 2 kg 040 g | 2 kg 040.1 g | 2 kg 040.1 g | 2 kg 040.4 g |
| Powdered cellulose | 20.3 | 2 kg 436 g | 2 kg 436 g | 2 kg 436 g | 2 kg 436 g |

(continued)

| Ingredient | Unit Formula (mg/Tab.) | Lot Quantity | Actual Lot Quantity | | |
|---|---|---|---|---|---|
| | | | Lot No. 001 | Lot No. 002 | Lot No. 003 |
| **Immediate Release layer** | | | | | |
| Sodium Starch Glycolate | 5.05 | 606 g | 606.03 g | 606.02 g | 606.02 g |
| Pregelatinized Corn Starch | 5.05 | 606 g | 606.04 g | 606.05 g | 606 g |
| Corn Starch | 20.3 | 2 kg 436 g | 2 kg 436 g | 2 kg 436.1 g | 2 kg 436.1 g |
| Mg Stearate | 1.65 | 198 g | 198.04 g | 198.03 g | 198.01 g |
| Purified Water* | - | 30 kg 754 g | 30 kg 754 g | 30 kg 754 g | 30 kg 754 g |
| Weight IR | | | 329.4 mg/Tab. | | 39.5 kg/lot |
| **Extended Release layer** | | | | | |
| APAP | 390.0 | 46 kg 800 g | 46 kg 800 g | 46 kg 800 g | 46 kg 800 g |
| Tramadol HCl, | 58.0 | 6 kg 960 g | 6 kg 960 g | 6 kg 960g | 6 kg 960 g |
| Hypromellose 2910, 15 mPas (HPMC E15) | 10.0 | 1 kg 200 g | 1 kg 200.14 g | 1 kg 200.1 g | 1 kg 200.10 g |
| Lambdacarrageenan (VISCARIN 109) | 72.5 | 8 kg 700 g | 8 kg 700 g | 8 kg 700.1 | 8 kg 700.2 g |
| Hypromellose 2208,2903 mPas (HPMC K4M) | 30.0 | 3 kg 600 g | 3 kg 600 g | 3 kg 600 | 3 kg 600 g |
| Polyethylene Oxide (POLYOX WSR N12K) | 30.0 | 3 kg 600 g | 3 kg 600.1 g | 3 kg 600.1 g | 3 kg 600.1 g |
| Mg Stearate | 5.96 | 715.2 g | 715.2 g | 715.2 g | 715.2 g |
| Purified Water* | - | 2 kg 880 g | 2 kg 880 g | 2 kg 880 g | 2 kg 880 g |
| Dehydrated Ethanol** | - | 4 kg 320 g | 4 kg 320 g | 4 kg 320 g | 4 kg 320.3 g |
| Weight ER | | | 596.5 mg/Tab. | | 71.6 kg/lot; ER+IR=111.1 kg/lot |
| **Coating layer** | | | | | |
| OPADRY yellow YS-1-6370-G *** | 25.0 | 3 kg 600 g | 3 kg 600 g | 3 kg 600 g | 3 kg 600 g |
| Carnauba wax | 0.041 | 4. 92 g | 4.92 g | 4.92 g | 4. 92 g |

(continued)

| Coating layer | | | | | |
|---|---|---|---|---|---|
| Purified Water**** | - | 25 kg 168 g | 25 kg 168 g | 25 kg 168 g | 25 kg 168 g |

* Water is removed during the drying process, and does not appear in the final product.
* Water is removed during the granulation process, and does not appear in the final product.
** Ethanol is removed during the granulation process, and does not appear in the final product.
*** Value was adjusted in consideration of loss during coating process. Actual amount needed for this lot include 20% excess allowance (3 kg-----→ 3.6 kg).
**** Water is removed during the coating process, and does not appear in the final product.

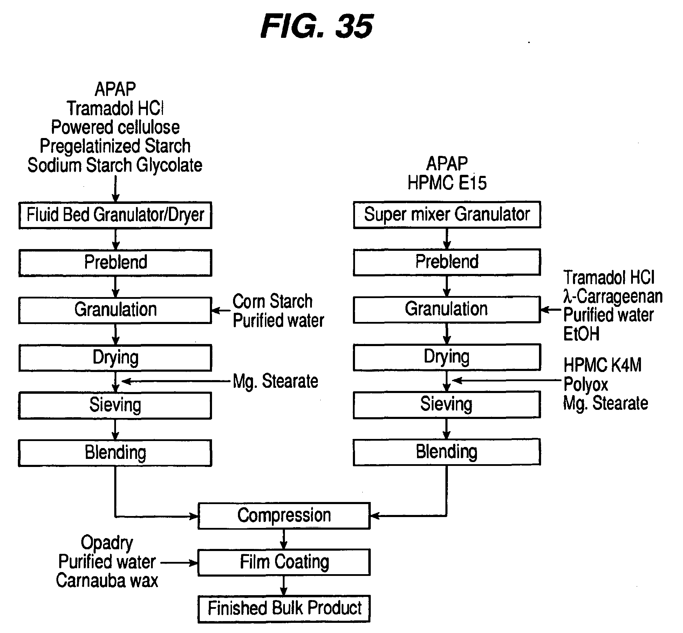

A fluid bed granulation manufacturing process was used for the IR layer, and a high-shear mixer granulation process was used for making the ER layer, drying, sieving and blending steps with subsequent compression. The compressed tablets were finally film-coated. The major equipment used during the manufacture is outlined as follows: granulation: high shear mixer granulator, fluid bed granulator; drying: fluid bed granulator; milling: oscillating sieve; blending: V-blender; tabletting machine: TMI double layer compress; coating: Hi-coater. The flow chart of the manufacturing process for the tablets is shown in FIG. 35.

In the preparation of IR granules, first a binder solution was prepared. The IR materials (APAP, tramadol HCl, powdered cellulose, pregelatinzed starch, sodium starch glycolate) were transferred into the fluid bed granulator and preblended. Granules of the materials were formed using the fluid bed granulator by spraying the required amount of binder solution into the material. The granules were dried and then passed along with magnesium stearate through a sieving mill machine to achieve desirable particle size. The resultant IR granules were blended using a V blender. In the preparation of ER granules, tramadol HCl was dissolved in 60% ethanol solution and lambda carrageenan was added to form the complex. APAP and HPMC E15 were preblended in a SuperMixer Granulator. The tramadol complex paste and the APAP/HPMC E15 were granulated together using a high-shear mixer. The wet granules were passed through a sieving machine to achieve desirable particle size. The granules were dried in a fluid-bed drier. The dried granules, along with the other agents (HPMC K4M, POLYOX) and magnesium stearate were passed through a sieving machine and then blended to form the ER blend. The IR blend and the ER blends were compressed into tablets at a weight of about 925.8 mg using an appropriate double-layer tablet press (e.g. TMI double layer press or equivalent) with embossed tablet tooling (49 sets upper, lower and die). Three batches (lots) of tablets were made. The dimension characteristics of the punches used in the tooling for making the tablets were: length: 19.05 mm; width: 7.62 mm; curve radius: 5.5 mm. The coating fluid (liquid) was made by mixing the appropriate amount of OPADRY Yellow YS-1-6370-G into purified water. Tablets to be coated (core tablets) were loaded in a coating pan. The core tablets were heated in the coating pan and coated with the coating fluid using an appropriate coater (e.g. Hi-coater or equivalent). After spraying was completed the pan was kept rotating to ensure drying of the tablets. Carnauba wax was sprinkled across the rotating tablet bed. The coating fluid can be a solution in which all ingredients are well solubilized in the solvent, or it can contain some particulate ingredients dispersed in the fluid. Coating fluids are well known in the art and those skilled in the art will know what alternatives can be used based on the disclosed examples disclosed herein.

The major equipment used during the manufacture of the tablets is outlined as follows:

1. Granulation: High Shear Mixer Granulator (Supermixer : 30 kg) Fluid Bed Granulator (Glatt WSG 30 : 30kg)
2. Drying: Fluid Bed Granulator (Glatt WSG 30: 30 kg)
3. Milling: Oscillating sieve
4. Blending: V-blender (100L)
5. Tabletting Machine: TMI compress
6. Coating : Pan-coater (30 kg)

Table 10 shows the parameters of the above equipment used in the manufacturing of the tablets in Lots 001, 002, and 003. In the drying process, the tablets were dried to a target weight percent moisture after drying (MafD) of 1 wt% to 3 wt%. A person skilled in the art will know how to use the above equipment in the manufacturing of the tablets under conditions of the parameters of Table 10. In Table 10, the values of the set-up parameters were applied to each lot and might vary slightly (as shown in the table).

Table 10 Process parameters of equipment

| Process | | Major Equipment | Process Parameters | | | | |
|---|---|---|---|---|---|---|---|
| | | | Items | Set-Up | Actual | | |
| | | | | | Lot 001 | Lot 002 | Lot 003 |
| High Shear granulate | Pre-blend | Supermixer | Impeller Speed<br>Time | 472 rpm<br>5 min | 472 rpm<br>5 min | 472 rpm<br>5 min | 472 rpm<br>5 min |
| | Granulate | | Impeller Speed<br>Mixing Time<br>End Ampere<br>Additional Amount of Ethanolic solution. | 472 rpm<br>35sec<br>13.7A<br><br>N/A ml | 472 rpm<br>35sec<br>13.7A<br><br>N/A ml | 472 rpm<br>35sec<br>13.8A<br><br>N/A ml | 472 rpm<br>35sec<br>13.8A<br><br>N/A ml |
| Dry | | Glatt WSG 30 | Inlet air flow (CFM)<br>Inlet Temp.<br>Outlet Temp. | 1500-2000<br>55-65°C (60 °C)<br>40-50°C | 60°C<br>47°C | 60°C<br>47°C | 60°C<br>47°C |
| | | | Shaking interval<br>Shaking Duration<br>End of Drying Time | 1 min<br>10 sec<br>MafD: 1.0~3.0% | 1.90% 50 min | 1.95% 51 min | 1.99% 50 min |
| Mill | | Fitz-mill | Speed<br>Screen Size | Medium 1.5 mm | Medium 1.5 mm | Medium 1.5 mm | Medium 1.5 mm |
| Final Blend | | V-mixer | Mixing time<br>Mixing Speed | Time 15min<br>14rpm | Time 15min<br>14rpm | Time 15min<br>14rpm | Time 15min<br>14rpm |
| Compress | | TMI compr ess No.1 or 2 | Machine Speed<br>No. of Stations<br>Punch size<br>Tablet weight | 32 rpm<br>49 st<br>19.05/7.62 mm<br>925.81 mg ±5% | 32 rpm<br>49 st<br>same<br>same | 32 rpm<br>49 st<br>same<br>same | 32 rpm<br>49 st<br>same<br>same |
| | Pre-heating | | Inlet Temp.<br>Outlet Temp.<br>Time | 70-80°c (75°C)<br>40-50°C (45 °C)<br>20-30 min | 80°C<br>50°C<br>20 min | 80°C<br>50°C<br>20 min | 80°C<br>50°C<br>20 min |
| | | | Rotational speed | 4 - 6 rpm | 5rpm | 5rpm | 5rpm |

(continued)

| Process | | Major Equipment | Process Parameters | | | | |
|---------|---|-----------------|-------|--------|------|------|------|
| | | | Items | Set-Up | Actual | | |
| | | | | | Lot 001 | Lot 002 | Lot 003 |
| Film-Coat | Coating | Pan-coater | Inlet Temp. | 70-80°C (75 °C) | 80°C | 80°C | 80°C |
| | | | Outlet Temp. | 40-50°C (45 °C) | 50°C | 50°C | 50°C |
| | | | No. of Spray gun | 2 ea | | | |
| | | | Nozzle Diameter | 1.0 mm | | | |
| | | | Distance | 15-20 cm | | | |
| | | | Spraying rate | 160-200 g/min | 180g/ min | 180g/ min | 180g/ min |
| | | | Spraying pressure Time | 4 bar 80 - 130 min | 4bar 141min | 4bar 146min | 4bar 148min |

Table 11 shows the particle size distribution in mesh of the immediate release (IR) granules used in Lots 001, 002, and 003 for the extended release tablets.

Table 11 Particle size distribution (in wt%) of IR particles

| | Mesh | Lot 001 | Lot 002 | Lot 003 |
|---|------|---------|---------|---------|
| Particle Size Distribution (wt%) | #18 | 2.21 | 0.42 | 0.18 |
| | #20 | 0.97 | 0.41 | 0.60 |
| | #35 | 12.57 | 4.57 | 6.36 |
| | #60 | 47.07 | 45.85 | 30.43 |
| | #100 | 15.97 | 23.57 | 24.38 |
| | #140 | 7.43 | 2.54 | 12.83 |
| | #200 | 9.91 | 12.25 | 9.85 |
| | pan | 3.86 | 10.41 | 15.38 |

The weight of the final tablets was about 951 mg per tablet. The weight of tablets manufactured was about 114 Kg per lot.

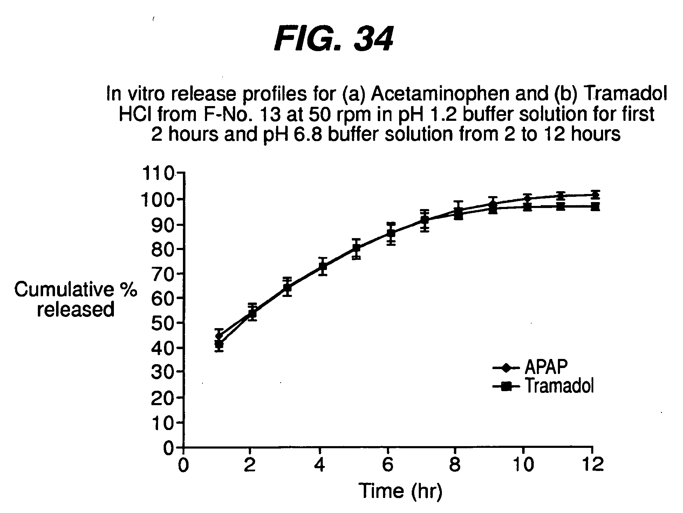

In the above F-No. 13 tablet, the IR layer is about 3.14 mm thick, and the ER layer was about 3.82 mm thick, with a total thickness of 6.96 mm. Under the above condition, the mean value of hardness for uncoated tablet was $8.5 \pm 1$ KP and the friability was less than 1% (0.23%). FIG. 34 shows dissolution profiles for the F-No. 13 (the coated tablets). The diamond data points represent the APAP data. The square data points represent the tramadol data. The value for relative standard deviation (CV) was less than 7% for all points measured (n=6). Beginning from the first hour through the twelveth hour, the wt% cumulative release of APAP was very close (less than 10% difference) to that of the tramadol. Starting from the second hour through the eighth hour, the difference was less than 5%. The result shows that a multiple layered dosage form was made that could provide cooridinated release of APAP and tramadol. In this embodiment, the release rates of tramadol and APAP were very close. The ratios of $T_{60}$, $T_{70}$, $T_{80}$, $T_{90}$ of APAP versus tramadol is less than 2, in fact less than 1.5 and is substantially close to 1. From the results of the release rate experiments it is clear that in a bilayer tablet the IR layer would disintegrate and release the drugs quickly (in a matter of minutes, such as 15 minutes). The drug release time in the IR layer is extremely short compared with the ER layer release, which takes 8 hours or more. Thus, it is reasonable to assume the release rate of drugs in the ER layer in the bi-layer tablet would be similar to that of an ER layer in *in vitro* dissolution tests in which only the ER layer was tested. Since the ER layer in F-No. 13 is almost identical to that of F-No. 7, the release exponent *n* would be about 0.75 for APAP and 0.6 for tramadol in the ER layer.

It has been found that complexing tramadol with an anionic polymer, preferably carrageenan to form an extended release layer in a tablet provides non-Fickian and/or Case II erosion controlled release, thus enabling the coordinated release with APAP. For comparison of the performance of difference tablets, the determination of MDT, $T_{80}$, and release exponent $n$ in the Korsmeyer equation is preferably done by *in vitro* experiments using the USP II (paddle) apparatus with the following method. The paddle position is 25 mm from the inside bottom of the vessel. The dissolution media is pH 6.8 phosphate buffer solution prepared according to USP method (USP SIF without enzyme) and the dissolution is done at 50 rpm/900ml at $37 \pm 0.5°C$. The dissolution media sample is to be taken at regular intervals to be filtered by $0.45\mu$ membrane filter and the concentrations of both tramadol HCl and APAP in the release medium is measured by an HPLC using an aqueous buffer solution / methanol solution as mobile phase. The mobile phase (pH 2.7 buffer: Methanol = 73 : 27) is to be filtered through a 0.45-um Millipore Filter (HAWP 04700) or equivalent and degassed by helium sparging. A dissolution Standard (100%), 37.5/325mg is made by accurately weighing 36.11/purity mg($\pm 1\%$) of acetaminophen into a 50 ml volumetric flask, transferring 10.0 ml of tramadol hydrochloride Stock Solution, dissolving and diluting to volume with pH 6.8 phosphate buffer. The tramadol hydrochloride Stock Solution is made by weighing 41.66/purity mg ($\pm 1\%$) of tramadol hydrochloride into a 100 ml volumetric flask, dissolve it and diluting to volume with pH 6.8 phosphate buffer. The HPLC column is SUPELCO LC-8-DB 150 x 4.6 mm; 5$\mu$m. Injection volume is 10 $\mu$l and flow rate is 2.5ml/min with run time of 16 minutes; retention time for APAP: approximately 1.2 min; and retention time for tramadol hydrochloride: approximately 4.0 min. The detector is Waters 490 UV programmable detector or equivalent (APAP 280 nm - 1.0 AUFS; tramadol hydrochloride 215 nm - 0.5 AUFS). Column temperature is about 35°C. USP II method is a standardized method. One skilled in the art can refer to the pharmacopeia for the USP II method.

The calculation of the percentage of the label (La, specified) amount of the drug in the sample can be calculated as

$$\% \text{ La Dissolved} = \frac{A_{sam} \times C_{std}}{A_{std} \times C_{t100}} \times 100$$

Where $A_{saro}$ = Tramadol hydrochloride or acetaminophen peak area for the sample,

$A_{std}$ = Tramadol hydrochloride or acetaminophen peak area for the standard,

$C_{std}$ = Standard concentration in mg/ml,

$C_{t100}$ = Theoretical 100% concentration in mg/ml,

La = Label amount of tramadol hydrochloride or APAP

With the present invention, regarding the ER layer, we *were* able to obtain release exponent $n$ in the Korsmeyer equation for tramadol at about above 0.45, even above 0.7, and even above 0.85. Preferably, the release exponent $n$ for APAP is about 0.46 to 1, more preferably about 0.6 to 0.9, more preferably about 0.6 to 0.8. Preferably, the release exponent $n$ for tramadol is about 0.46 to 0.7, more preferably about 0.5 to 0.7, more preferably 0.5 to 0.65.

We were also able to achieve ratios of $T_{80}$ of APAP versus tramadol at values close to 1 in the bilayer tablet. Preferably, the $T_{80}$ ratio is about below 2, preferably about below 1.5 and more preferably about between 1.5 and 1. It is more preferred that the $T_{80}$ ratio is between 0.9 and 1.1. It is also preferred that $T_{80}$ is about from 8 to 12 hours, more preferably about from 10 to 12 hours. Table 12 shows the the $T_{80}$ data for F-No. 13.

Table 12 $T_{80}$ data for F-No. 13 tablets

| Time | 1 | 2 | 3 | 4 | 5 | 6 | 7 | 8 | 9 | 10 | 11 | 12 |
|------|-----|-----|-----|-----|-----|-----|-----|-----|-----|-----|-----|-----|
| T80 | 1.096 | 1.009 | 1.007 | 0.997 | 0.995 | 0.998 | 1.002 | 1.010 | 1.021 | 1.032 | 1.042 | 1.047 |

*In vivo* extended release of bi-layer tablet

Extended release tablets (made in accordance with the pilot plant formulation described in Table 9) were compared with an established branded formulation tramadol/APAP combination (ULTRACET) in healthy male volunteers in Korea on the relative bioavailability and other pharmacokinetic properties. An ULTRACET tablet contains 37.5 mg tramadol hydrochloride and 325 mg APAP. Such ULTRACET tablets are available commercially. Inactive ingredients in the tablet are powdered cellulose, pregelatinized corn starch, sodium starch glycolate, starch, purified water, magnesium stearate, OPADRY® Light Yellow, and carnauba wax. The labeling description and use of ULTRACET tablets can be found in the labeling describing this patch and its use in, e.g., USFDA NDA No. 021123 (the label approved on April 16, 2004, ©OMP 2003), which is incorporated by reference herein it its entirety.

A randomized, multiple-dose, two-treatment, two-period, two-sequence, crossover study was performed in healthy male Korean volunteers under fasting conditions with a washout of 4 days between the study periods as shown in the following Table 13.

Table 13

| Sequence | N (individuals) | First period(4 days) | Second period(4 days) |
|---|---|---|---|
| Sequence 1 (AB) | 6 | ULTRACET (A) | ER tablet (B) |
| Sequence 2 (BA) | 6 | ER tablet (B) | ULTRACET (A) |

After screening, at the start of the sequence of drug administration, each individual was administered the selected drug according to the First period for 4 days, followed by 4 days of washout without drug administration, and then followed by 4 days of drug administration according to the second period. The individuals were followed up for 4 days post-drug-administration to record the data for the blood samples of the individuals. During the drug administration sequences, commercial ULTRACET tablets (designated as A in Table 13 and the ER tablets (designated as B in Table 13) were orally administered 14 times at 6 hr intervals, and 7 times at 12 hr intervals, respectively, according to Table 13. Blood samples were collected according to pre-determined time intervals after the dose.

The data of Table 13 were used to determine the bioavailability of the drugs in the tablets. As used herein, the term "bioavailability", refers to the rate and extent to which the active ingredient or active moiety is absorbed from a drug product and becomes available at the site of action. The rate and extent are established by the pharmacokinetic-parameters, such as, the peak blood or plasma concentration ($C_{max}$) of the drug and the area under the blood or plasma drug concentration-time curve (AUC).

In pharmacokinetics, the term "AUC" means the area under the curve obtained in a subject by plotting serum concentration of the beneficial agent in the subject against time, as measured from the time of start of dosing, to a time "t" after the start of dosing. For steady state drug administration, the $AUC_{ss}$ is the area under the curve for a dosing period with doses administered periodically to time infinite. The AUC can be obtained by assaying serum samples from a patient.

As used herein, the term "$C_{max}$" refers to the peak blood or plasma concentration of the drug. The time "$t_{max}$" refers to the time to reach peak blood or plasma concentration of the drug. The term "$t_{1/2}$" is half life and refers to the time it takes for the plasma concentration of the drug to decay by half.

Plasma APAP/Tramadol concentrations were determined using a validated LC/MS/MS method. A plasma concentration-time curve was generated for each volunteer from which the primary parameters ($C_{max}$, $T_{max}$, $AUC_{0-12hr}$) at the first day after the dose and the secondary parameters ($C_{max(ss)}$, $T_{max(ss)}$, $AUC_{0-12h,ss}$, and $t_{1/2}$) at steady state were determined using noncompartmental analysis with WINNONLIN® 5.2.1 (Pharsight Co, CA, USA). Bioequivalence, for example, was defined using regulatory requirements set forth by Korea and US Food and Drug Administration (bioequivalence acceptance range, 0.80-1.25). To be bioequivalent to the commercial ULTRACET tablet, the 90% confidence interval (CI) of the ratio of the steady state mean $C_{max}$ of a new ER tablet to that the ULTRACET tablet of the same dose strength needs to be within 80% to 125% (i.e., 0.8 to 1.25) at a=0.05; and the 90% confidence interval (CI) of the ratio of mean $AUC_{ss}$ of a new ER tablet to that of the commercial ULTRACET needs to be within 80% to 125%.

A total of 12 volunteer individuals completed the study. The mean age of volunteers was 24.4 ± 5.2 years, and the mean body weight was 65.1 ± 6.0 kg. The mean (with SD) values of the pharmacokinetic parameters on tramadol after administration of the commercial ULTRACET tablets and the ER tablets of the present invention were shown in the Table 14 and Table 15 below.

Table 14. Pharmacokinetic parameters for Tramadol

| Parameters | ULTRACET (N=12) | | | ER (N=12) | | |
|---|---|---|---|---|---|---|
| | Mean | SD | CV (%) | Mean | SD | CV (%) |
| $T_{max}$ (h) | 1.0 [1.0-3.5][1] | | | 4.0 [2.0-6.0][1] | | |
| $C_{max}$ (μg/L) | 206.13 | 29.06 | 14.1 | 179.30 | 28.88 | 16.1 |
| $AUC_{0-12h}$ (μg*h/L) | 1380.1 | 207.6 | 15.0 | 1501.0 | 307.9 | 20.5 |
| $T_{max,ss}$ (h) | 1.0 [0.5-2.0][1] | | | 3.0 [1.0-4.0][1] | | |

(continued)

| Parameters | ULTRACET (N=12) | | | ER (N=12) | | |
|---|---|---|---|---|---|---|
| | Mean | SD | CV (%) | Mean | SD | CV (%) |
| $C_{max,ss}$ (µg/L) | 351.81 | 55.86 | 15.9 | 305.64 | 53.21 | 17.4 |
| $AUC_{0-12h,ss}$ (µg*h/L) | 2789.0 | 507.7 | 18.2 | 2638.7 | 469.1 | 17.8 |
| $t_{1/2}$ (h) | 7.08 | 1.94 | 27.4 | 7.01 | 0.96 | 13.7 |
| [1] median [minimum-maximum] | | | | | | |

Table 15. Comparison of $C_{max,ss}$, $AUC_{0-12h,ss}$ for Tramadol

| Parameters | ULTRACET (N=12) | ER (N=12) | Difference of Geometric mean (90% CI) | Geometric Mean Ratio [3] (90% CI) |
|---|---|---|---|---|
| $C_{max,ss}$ (µg/L) | 351.81 ± 55.86[1] | 305.64 ± 53.21[1] | -0.144 (-0.227 - 0.061) | 0.87 (0.80 - 0.94) |
| | 5.85 ± 0.15[2] | 5.71 ± 0.18[2] | | |
| $AUC_{0-12h,ss}$ (µg*h/L) | 2789.0 ± 507.7[1] | 2638.7 ± 469.1[1] | -0.054 (-0.094 - -0.014) | 0.95 (0.91 - 0.99) |
| | 7.92 ± 0.18[2] | 7.86 ± 0.17[2] | | |

[1] Arithmetic mean ± standard deviation

[2] Logarithmic transformed geometric mean ± standard deviation

[3] Geometric mean ratio of ER to ULTRACET. Arithmetic values were obtained from actual individual data. However, bioequivalence is decided by the difference of geometric mean at 90% confidence interval, so geometric means were converted from arithmetic means.

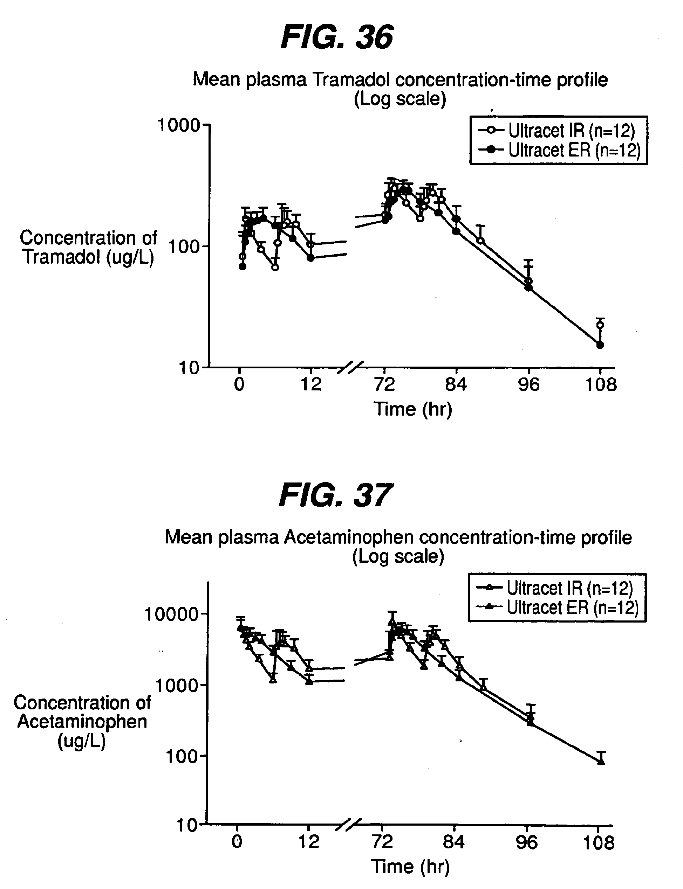

FIG. 36 shows in portion the mean plasma concentration-time profiles of tramadol after multiple oral administrations of ULTRACET tablets and ER tablets of the present invention. The bars in the graph represent standard deviations. The curve with the solid disks data points represent the ER data, showing peaks about every 12 hours. The curve with the circle data points represent the ULTRACET data, showing peaks about every 6 hours.

The mean (with SD) values of the pharmacokinetic parameters on APAP after administration of the commercial ULTRACET tablets and the ER tablets of the present invention were shown in the Table 16 and Table 17 below.

Table 16. Pharmacokinetic parameters for APAP

| Parameters | ULTRACET (N=12) | | | ER (N=12) | | |
|---|---|---|---|---|---|---|
| | Mean | SD | CV (%) | Mean | SD | CV (%) |
| $T_{max}$ (h) | 0.5 [0.5-1.5][1] | | | 0.5 [0.5 - 2.0] [1] | | |
| $C_{max}$ (µg/L) | 7388.1 | 2022.7 | 27.4 | 6574.8 | 1100.4 | 16.7 |
| $AUC_{0-12h}$ (µg*h/L) | 33780.6 | 6262.5 | 18.5 | 35294.3 | 7222.9 | 20.5 |
| $T_{max,ss}$ (h) | 0.5 [0.5-1.5][1] | | | 0.5 [0.5 - 2.0][1] | | |
| $C_{max,ss}$ (µg/L) | 8180.8 | 2025.1 | 24.8 | 6853.9 | 1290.0 | 18.8 |
| $AUC_{0-12h,ss}$ (µg*h/L) | 42635.0 | 8711.2 | 20.4 | 40394.3 | 10127.7 | 25.1 |
| $t_{1/2}$ (h) | 5.21 | 1.01 | 19.4 | 6.67 | 2.37 | 35.5 |
| [1] median [minimum - maximum] | | | | | | |

Table 17. Comparison of $C_{max,ss}$, $AUC_{0-12h,ss}$ for APAP

| Parameters | ULTRACET (N=12) | ER (N=12) | Difference of Geometric mean (90% CI) | Geometric Mean Ratio [3] (90% CI) |
|---|---|---|---|---|
| $C_{max,ss}$ ($\mu$g/L) | 8180.8 $\pm$ 2025.1 [1] | 6853.9 $\pm$ 1290.0 [1] | -0.164 (-0.270- -0.059) | 0.85 (0.76 - 0.94) |
| | 8.98 $\pm$ 0.26 [2] | 8.82 $\pm$ 0.19 [2] | | |
| $AUC_{0-12h,ss}$ ($\mu$g*h/L) | 42635.0 $\pm$ 8711.2 [1] | 40394.3 $\pm$ 10127.7 [1] | -0.065 (-0.119- -0.011) | 0.94 (0.89 - 0.99) |
| | 10.64 $\pm$ 0.23 [2] | 10.57 $\pm$ 0.28 [2] | | |
| [1] Arithmetic mean $\pm$ standard deviation [2] Logarithmic transformed geometric mean $\pm$ standard deviation [3] Geometric mean ratio of ER to ULTRACET | | | | |

FIG. 37 shows in portion the mean plasma concentration-time profiles of APAP after multiple oral administrations of ULTRACET tablets and ER tablets of the present invention. The bars in the graph represent standard deviations. The curve with the solid disks data points represent the ER data, showing peaks about every 12 hours. The curve with the circle data points represent the ULTRACET data, showing peaks about every 6 hours.

The analysis of variance data of the above in vivo study, including the data of FIG. 36 and Fig. 37 showed no significant effect of formulation, period, or sequence on the studied pharmacokinetic parameters. The 90% CIs of the treatment ratios for the values of $C_{max,ss}$ and $AUC_{0-12h(ss)}$ were 0.87 and 0.95 for tramadol and 0.85 and 0.94 for APAP, respectively. All were within the standard bioequivalence acceptance range of 0.80 to 1.25. In this *in vivo* study in a selected population of healthy volunteers, the $C_{max,ss}$ and $AUC_{0-12h,ss}$ were not statistically significantly different between commercial ULTRACET tablets and new extended release formulation and these were found to be bioequivalent. Further, both formulations were well tolerated. No adverse events were reported in this study. Therefore, the new ER formulation of the present invention is shown to be bioequivalent in vivo to commercial ULTRACET tablets and therefore should be render effective and efficacious therapeutic effect for pain treatment on humans, in the same bioequivalent way as commercial ULTRACET tablets.

The practice of the present invention will employ, unless otherwise indicated, conventional methods used by those in pharmaceutical product development within those of skill of the art. Embodiments of the present invention have been described with specificity. The embodiments are intended to be illustrative in all respects, rather than restrictive, of the present invention. Further, where a substance is described to comprise certain ingredients, it is contemplated that a substance also be made consisting essentially of the ingredients.

## Claims

1. A pharmaceutical composition, comprising acetaminophen and a complexed tramadol material, that exhibits coordinated sustained release upon dissolution resulting in coordinated accumulative release of tramadol and accumulative release of acetaminophen over time, wherein the complexed tramadol material is complexed using carrageenan.

2. The composition according to Claim 1, wherein the complexed tramadol material is complexed using carrageenan, and tramadol salt

3. The composition according to Claim 1, wherein the sustained release is for a period of 4 to 12 hours over the whole period or over a period of 10 hours or more, for both tramadol and acetaminophen.

4. The composition according to Claim 1, wherein in the sustained release when the wt% accumulative release of tramadol is 40 wt%, the wt% accumulative release of acetaminophen is less than 25wt% different from the wt% accumulative release of tramadol.

5. The composition according to Claim 1, wherein in the sustained release starting from when the wt% accumulative release of tramadol is 40 wt%, the wt% accumulative release of acetaminophen is never more than 20wt% or never more than 10% different from the wt% accumulative release of tramadol.

6. The composition according to Claim 1, wherein in the sustained release after the first hour in a sustained release of at least 12 hours, the wt% accumulative release of acetaminophen is never more than 10wt% different from the wt% accumulative release of tramadol.

7. The composition according to Claim 1, wherein the sustained release accumulative releases are determined by United States Pharmacopeia Apparatus II (USP II) Paddle method at 37 °C at 50 rpm/900ml in vitro in a dissolution media of pH 6.8 simulated intestinal fluid without enzyme.

8. The composition according to claim 1, the composition comprising a layer of an extended release composition attached to an immediate release layer, the extended release composition including acetaminophen and the complexed tramadol material, the immediate release layer including acetaminophen and tramadol material that is mostly uncomplexed.

9. The composition according to claim 1, the composition comprising a layer of an extended release composition attached to an immediate release layer, the extended release composition including disintegrant, acetaminophen and the complexed tramadol material, the complexed tramadol material is a complex of lambda carrageenan and tramadol HCl, the immediate release layer including hydrophilic polymeric retarding agent, acetaminophen and tramadol material that is mostly uncomplexed.

10. The composition according to Claim 9, wherein the hydrophilic polymeric retarding agent is selected from the group comprising polysaccharide or derivative thereof, agar, agarose, gum; and the extended release composition includes hydroxypropyl methyl cellulose and filler.

11. The composition according to claim 1, the composition comprising a layer of an extended release composition adjacent to an immediate release layer, the extended release composition including disintegrating carrier, acetaminophen and the complexed tramadol material, the complexed tramadol material is a complex of lambda carrageenan and tramadol HCl, the immediate release layer including hydrophilic polymeric retarding agent, acetaminophen and tramadol material that is mostly uncomplexed.

12. The composition according to Claim 11, wherein in the extended release composition the weight ratio of acetaminophen to tramadol material in complexed tramadol material is from 1: 1 to 20:1, or from 5:1 to 10:1.

13. The composition according to Claim 1, wherein the pharmaceutical composition comprising acetaminophen and a complexed tramadol material is a layer and both acetaminophen and tramadol in the layer are released at a non-Fickian manner.

14. The composition according to Claim 1, wherein the pharmaceutical composition comprising acetaminophen and a complexed tramadol material is a layer and both acetaminophen and tramadol in the layer are released at a manner with a release exponent $n$ of about 0.5 to 0.7 for tramadol and a release exponent $n$ of 0.6 to 0.9 for Acetaminophen in Korsmeyer equation.

15. The composition according to Claim 1, wherein the pharmaceutical composition comprising acetaminophen and a complexed tramadol material is a layer and both acetaminophen and tramado 1 in the layer are released at a manner that the ratio of $T_{80}$ of acetaminophen to $T_{80}$ of tramadol is between 0.9 to 1.1 with a $T_{80}$ of 8 hours or more.

16. A method of making a dose form of a pharmaceutical composition, comprising forming a complexed tramadol material; and
forming a compacted form including the complexed tramadol material and acetaminophen, wherein the compacted form exhibits coordinated sustained release upon dissolution in use resulting in coordinated accumulative release of tramadol and accumulative release of acetaminophen over time, and wherein the complexed tramadol material is complexed using carrageenan .

17. The method according to Claim 16, comprising using a tramadol salt and carrageenan to form the complexed tramadol material.

**18.** The method according to Claim 16, comprising using a tramadol salt and carrageenan to form the complexed tramadol material as a paste, drying the paste and forming granules therefrom.

**19.** The method according to Claim 16, comprising using a tramadol salt and carrageenan to form the complexed tramadol material as a paste, drying the paste, forming granules therefrom and compacting the granules to form the compacted form.

**20.** The method according to Claim 16, comprising using a tramadol salt and lambda carrageenan to form the complexed tramadol material, forming granules therefrom, compacting the granules to form the compacted form, and forming an additional layer over said compacted form, the additional layer including hydrophilic polymeric retarding agent, acetaminophen and a tramadol material that is mostly uncomplexed.

**21.** The method according to Claim 20, comprising using a weight ratio from 1: 1 to 20:1 or from 5:1 to 10:1, for acetaminophen to the tramadol material to form the compacted form.

**22.** The method according to Claim 20, such that in the sustained release when the wt% accumulative release of tramadol is 40 wt%, the wt% accumulative release of acetaminophen is less than 25wt% different from the wt% accumulative release of tramadol.

**23.** The method according to Claim 20, wherein in the sustained release starting from when the wt% accumulative release of tramadol is 40 wt%, the wt% accumulative release of acetaminophen is never more than 20wt% different from the wt% accumulative release of tramadol.

**24.** The method according to Claim 16, comprising using at least two different kind of hydroxypropylmethyl cellulose in making the compacting form.

**25.** A complexed tramadol material and acetaminophen for use in a method of treating pain, wherein said complexed tramadol material and acetaminophen, exhibits coordinated sustained release of said tramadol and acetaminophen upon oral administration in a patient resulting in coordinated accumulative release of tramadol and accumulative release of acetaminophen over time, and wherein the complexed tramadol material is complexed using carrageenan.


**Patentansprüche**

**1.** Pharmazeutische Zusammensetzung, umfassend Acetaminophen und ein komplexiertes Tramadol-Material, das koordinierte verzögerte Freisetzung nach Auflösung zeigt, was zu koordinierter kumulativer Freisetzung von Tramadol und kumulativer Freisetzung von Acetaminophen im Zeitverlauf führt, wobei das komplexierte Tramadol-Material unter Verwendung von Carrageen komplexiert wird.

**2.** Zusammensetzung nach Anspruch 1, wobei das komplexierte Tramadol-Material unter Verwendung von Carrageen und Tramadolsalz komplexiert wird.

**3.** Zusammensetzung nach Anspruch 1, wobei die verzögerte Freisetzung für einen Zeitraum von 4 bis 12 Stunden über den gesamten Zeitraum oder über einen Zeitraum von 10 Stunden oder mehr, sowohl für Tramadol als auch für Acetaminophen, besteht.

**4.** Zusammensetzung nach Anspruch 1, wobei in der verzögerten Freisetzung, wenn die kumulative Freisetzung, in Gew.-%, von Tramadol 40 Gew.-% beträgt, die kumulative Freisetzung, in Gew.-% von Acetaminophen sich um weniger als 25 Gew.-% von der kumulativen Freisetzung, in Gew.-%, von Tramadol unterscheidet.

**5.** Zusammensetzung nach Anspruch 1, wobei in der verzögerten Freisetzung, beginnend ab da, wenn die kumulative Freisetzung, in Gew.-%, von Tramadol 40 Gew.-% beträgt, die kumulative Freisetzung, in Gew.-% von Acetaminophen sich niemals mehr als 20 Gew.-% oder niemals mehr als 10% von der kumulativen Freisetzung, in Gew.-%, von Tramadol unterscheidet.

**6.** Zusammensetzung nach Anspruch 1, wobei in der verzögerten Freisetzung nach der ersten Stunde in einer verzögerten Freisetzung von wenigstens 12 Stunden, die kumulative Freisetzung, in Gew.-%, von Acetaminophen sich niemals mehr als 10 Gew.-% von der kumulativen Freisetzung, in Gew.-%, von Tramadol unterscheidet.

7. Zusammensetzung nach Anspruch 1, wobei die verzögerten kumulativen Freisetzungen mit der US-Pharmakopöe Gerät II (USP II) Paddelmethode bei 37°C und 50 U/min/900 ml in vitro in einem Auflösungsmedium von pH 6,8, simuliert in Darmflüssigkeit ohne Enzym, bestimmt werden.

8. Zusammensetzung nach Anspruch 1, wobei die Zusammensetzung eine Schicht aus einer Zusammensetzung mit verlängerter Freisetzung, die an einer Schicht mit sofortiger Freisetzung befestigt ist, umfasst, wobei die Zusammensetzung mit verlängerter Freisetzung Acetaminophen und das komplexierte Tramadol-Material aufweist, wobei die Schicht mit sofortiger Freisetzung Acetaminophen und das Tramadol-Material aufweist, das größtenteils unkomplexiert ist.

9. Zusammensetzung nach Anspruch 1, wobei die Zusammensetzung eine Schicht aus einer Zusammensetzung mit verlängerter Freisetzung, die an einer Schicht mit sofortiger Freisetzung befestigt ist, umfasst, wobei die Zusammensetzung mit verlängerter Freisetzung ein Sprengmittel, Acetaminophen und das komplexierte Tramadol-Material aufweist, wobei das komplexierte Tramadol-Material ein Komplex aus Lambda-Carrageen und Tramadol-HCl ist, wobei die Schicht mit sofortiger Freisetzung ein hydrophiles polymeres Verzögerungsmittel, Acetaminophen und größtenteils unkomplexiertes Tramadol-Material aufweist.

10. Zusammensetzung nach Anspruch 9, wobei das hydrophile polymere Verzögerungsmittel aus der Gruppe ausgewählt ist, die Polysaccharid oder ein Derivat davon, Agar, Agarose, Gummi umfasst; und die Zusammensetzung mit verlängerter Freisetzung Hydroxypropylmethylcellulose und Füllstoff aufweist.

11. Zusammensetzung nach Anspruch 1, wobei die Zusammensetzung eine Schicht aus einer Zusammensetzung mit verlängerter Freisetzung neben einer Schicht mit sofortiger Freisetzung umfasst, wobei die Zusammensetzung mit verlängerter Freisetzung einen zerfallenden Träger, Acetaminophen und das komplexierte Tramadol-Material aufweist, wobei das komplexierte Tramadol-Material ein Komplex aus Lambda-Carrageen und Tramadol-HCl ist, wobei die Schicht mit sofortiger Freisetzung ein hydrophiles polymeres Verzögerungsmittel, Acetaminophen und größtenteils unkomplexiertes Tramadol-Material aufweist

12. Zusammensetzung nach Anspruch 11, wobei in der Zusammensetzung mit verlängerter Freisetzung das Gewichtsverhältnis von Acetaminophen zu Tramadol-Material in komplexiertem Tramadol-Material 1:1 bis 20:1 oder 5:1 bis 10:1 beträgt.

13. Zusammensetzung nach Anspruch 1, wobei die Acetaminophen und ein komplexiertes Tramadol-Material umfassende pharmazeutische Zusammensetzung eine Schicht ist und sowohl Acetaminophen als auch Tramadol in der Schicht auf Nicht-Fick'sche Weise freigesetzt werden.

14. Zusammensetzung nach Anspruch 1, wobei die Acetaminophen und ein komplexiertes Tramadol-Material umfassende pharmazeutische Zusammensetzung eine Schicht ist und sowohl Acetaminophen als auch Tramadol in der Schicht auf eine Weise mit einem Freisetzungsexponenten $n$ von ungefähr 0,5 bis 0,7 für Tramadol und einem Freisetzungsexponenten $n$ von 0,6 bis 0,9 für Acetaminophen in Korsmeyer-Gleichung freigesetzt werden.

15. Zusammensetzung nach Anspruch 1, wobei die Acetaminophen und ein komplexiertes Tramadol-Material umfassende pharmazeutische Zusammensetzung eine Schicht ist und sowohl Acetaminophen als auch Tramadol in der Schicht auf eine Weise freigesetzt werden, dass das Verhältnis von $T_{80}$ von Acetaminophen zu $T_{80}$ von Tramadol zwischen 0,9 und 1,1 1 mit $T_{80}$ von 8 Stunden oder mehr beträgt.

16. Verfahren zur Herstellung einer Dosierform einer pharmazeutischen Zusammensetzung, umfassend die Bildung eines komplexierten Tramadol-Materials; und Bildung einer kompaktierten Form, einschließlich des komplexierten Tramadol-Materials und Acetaminophen, wobei die kompaktierte Form koordinierte verzögerte Freisetzung nach Auflösung im Gebrauch zeigt, was zu koordinierter kumulativer Freisetzung von Tramadol und kumulativer Freisetzung von Acetaminophen im Zeitverlauf führt, und wobei das komplexierte Tramadol-Material unter Verwendung von Carrageen komplexiert wird.

17. Verfahren nach Anspruch 16, umfassend ein Tramadolsalz und Carrageen zur Bildung des komplexierten Tramadol-Materials.

18. Verfahren nach Anspruch 16, umfassend die Verwendung eines Tramadolsalzes und von Carrageen zur Bildung des komplexierten Tramadol-Materials als Paste, Trocknen der Paste und Bildung eines Granulats daraus.

**19.** Verfahren nach Anspruch 16, umfassend die Verwendung eines Tramadolsalzes und von Carrageen zur Bildung des komplexierten Tramadol-Materials als Paste, Trocknen der Paste, Bildung eines Granulats daraus und Kompaktieren des Granulats zur Bildung der kompaktierten Form.

**20.** Verfahren nach Anspruch 16, umfassend die Verwendung eines Tramadolsalzes und von Lambda-Carrageen zur Bildung des komplexierten Tramadol-Materials, Bildung eines Granulats daraus, Kompaktieren des Granulats zur Bildung der kompaktierten Form, und Bildung einer zusätzlichen Schicht über der kompaktierten Form, wobei die zusätzliche Schicht ein hydrophiles polymeres Verzögerungsmittel, Acetaminophen und größtenteils unkomplexiertes Tramadol-Material aufweist.

**21.** Verfahren nach Anspruch 20, umfassend die Verwendung eines Gewichtsverhältnisses von 1:1 bis 20:1 oder von 5:1 bis 10:1 für Acetaminophen zum Tramadol-Material zur Bildung der kompaktierten Form.

**22.** Verfahren nach Anspruch 20, so dass in der verzögerten Freisetzung, wenn die kumulative Freisetzung, in Gew.-%, von Tramadol 40 Gew.-% beträgt, die kumulative Freisetzung, in Gew.-% von Acetaminophen sich um weniger als 25 Gew.-% von der kumulativen Freisetzung, in Gew.-%, von Tramadol unterscheidet.

**23.** Verfahren nach Anspruch 20, so dass in der verzögerten Freisetzung, beginnend ab da, wenn die kumulative Freisetzung, in Gew.-%, von Tramadol 40 Gew.-% beträgt, die kumulative Freisetzung, in Gew.-% von Acetaminophen sich niemals mehr als 20 Gew.-% von der kumulativen Freisetzung, in Gew.-%, von Tramadol unterscheidet.

**24.** Verfahren nach Anspruch 16, umfassend wenigstens zwei verschiedene Arten von Hydroxpropylmethylcellulose bei der Herstellung der kompaktierten Form.

**25.** Komplexiertes Tramadol-Material und Acetaminophen zur Verwendung in einer Methode zur Behandlung von Schmerzen, wobei das komplexierte Tramadol-Material und Acetaminophen koordinierte verzögerte Freisetzung von Tramadol und Acetaminophen bei oraler Verabreichung in einem Patienten zeigen, was zu koordinierter kumulativer Freisetzung von Tramadol und kumulativer Freisetzung von Acetaminophen im Zeitverlauf führt, und wobei das komplexierte Tramadol-Material unter Verwendung von Carrageen komplexiert wird.

## Revendications

**1.** Composition pharmaceutique comprenant de l'acétaminophène et une matière à base de tramadol complexé, qui présente une libération prolongée coordonnée lors de la dissolution, entraînant une libération cumulative coordonnée de tramadol et une libération cumulative d'acétaminophène au cours du temps, où la matière à base de tramadol complexé est complexée à l'aide de carraghénane.

**2.** Composition selon la Revendication 1, où la matière à base de tramadol complexé est complexée à l'aide de carraghénane et de sel de tramadol.

**3.** Composition selon la Revendication 1, où la libération prolongée se fait sur une durée de 4 à 12 heures au cours de la durée totale ou sur une durée de 10 heures ou plus, pour le tramadol et l'acétaminophène.

**4.** Composition selon la Revendication 1, où dans la libération prolongée, lorsque la libération cumulative en % massiques de tramadol est de 40 % en masse, la libération cumulative en % massiques d'acétaminophène est différente de moins de 25 % en masse de la libération cumulative en % massiques de tramadol.

**5.** Composition selon la Revendication 1, où dans la libération prolongée démarrant du point où la libération cumulative en % massiques de tramadol est de 40 % en masse, la libération cumulative en % massiques d'acétaminophène n'est jamais supérieure à 20 % en masse ou n'est jamais différente de plus de 10 % de la libération cumulative en % massiques de tramadol.

**6.** Composition selon la Revendication 1, où dans la libération prolongée après la première heure dans une libération prolongée d'au moins 12 heures, la libération cumulative en % massiques d'acétaminophène n'est jamais différente de plus de 10 % en masse de la libération cumulative en % massiques de tramadol.

**7.** Composition selon la Revendication 1, où les libérations cumulatives en libération prolongée sont déterminées par la Méthode II à pales de la pharmacopée des États-Unis (USP II) à 37 °C et 50 rpm/900 ml *in vitro* dans un milieu de dissolution de pH 6,8 de fluide intestinal simulé sans enzyme.

8. Composition selon la revendication 1, la composition comprenant une couche d'une composition à libération étendue fixée à une couche à libération immédiate, la composition à libération étendue incluant de l'acétaminophène et la matière à base de tramadol complexé, la couche à libération immédiate incluant de l'acétaminophène et une matière à base de tramadol qui est principalement non complexée.

9. Composition selon la revendication 1, la composition comprenant une couche d'une composition à libération étendue fixée à une couche à libération immédiate, la composition à libération étendue incluant un agent délitant, de l'acétaminophène et la matière à base de tramadol complexé, la matière à base de tramadol complexé étant un complexe de lambda-carraghénane et de tramadol HCl, la couche à libération immédiate incluant un agent retardateur polymère hydrophile, de l'acétaminophène et une matière à base de tramadol qui est principalement non complexée.

10. Composition selon la Revendication 9, où l'agent retardateur polymère hydrophile est choisi dans le groupe comprenant les polysaccharides ou leurs dérivés, l'agar, l'agarose, les gommes ; et la composition à libération étendue inclut de l'hydroxypropylméthylcellulose et une charge.

11. Composition selon la revendication 1, la composition comprenant une couche d'une composition à libération étendue adjacente à une couche à libération immédiate, la composition à libération étendue incluant un support délitant, de l'acétaminophène et la matière à base de tramadol complexé, la matière à base de tramadol complexé étant un complexe de lambda-carraghénane et de tramadol HCl, la couche à libération immédiate incluant un agent retardateur polymère hydrophile, de l'acétaminophène et une matière à base de tramadol qui est principalement non complexée.

12. Composition selon la Revendication 11, où dans la composition libération étendue, le rapport massique de l'acétaminophène sur la matière à base de tramadol dans la matière à base de tramadol complexé est compris entre 1:1 et 20:1, ou entre 5:1 et 10:1.

13. Composition selon la Revendication 1, où la composition pharmaceutique comprenant de l'acétaminophène et une matière à base de tramadol complexé est une couche et où l'acétaminophène comme le tramadol présents dans la couche sont libérés de façon non fickienne.

14. Composition selon la Revendication 1, où la composition pharmaceutique comprenant de l'acétaminophène et une matière à base de tramadol complexé est une couche et où l'acétaminophène comme le tramadol présents dans la couche sont libérés de sorte à présenter un exposant de libération n d'environ 0,5 à 0,7 pour le tramadol et un exposant de libération n de 0,6 à 0,9 pour l'acétaminophène dans l'équation de Korsmeyer.

15. Composition selon la Revendication 1, où la composition pharmaceutique comprenant de l'acétaminophène et une matière à base de tramadol complexé est une couche et où l'acétaminophène comme le tramadol présents dans la couche sont libérés de sorte que le rapport du $T_{80}$ de l'acétaminophène sur le $T_{80}$ du tramadol soit compris entre 0,9 et 1,1 avec un $T_{80}$ de 8 heures ou plus.

16. Procédé de fabrication d'une forme galénique d'une composition pharmaceutique, comprenant la mise en forme d'une matière à base de tramadol complexé ; et
la mise en forme d'une forme compactée incluant la matière à base de tramadol complexé et de l'acétaminophène, où la forme compactée présente une libération prolongée coordonnée lors de sa dissolution en cours d'utilisation, ce qui permet d'obtenir une libération cumulative coordonnée de tramadol et une libération cumulative d'acétaminophène au cours du temps, et où la matière à base de tramadol complexé est complexée en utilisant du carraghénane.

17. Procédé selon la Revendication 16, comprenant l'utilisation d'un sel de tramadol et de carraghénane pour former la matière à base de tramadol complexé.

18. Procédé selon la Revendication 16, comprenant l'utilisation d'un sel de tramadol et de carraghénane pour former la matière à base de tramadol complexé sous forme d'une pâte, le séchage de la pâte et la formation de granules à partir de celle-ci.

**19.** Procédé selon la Revendication 16, comprenant l'utilisation d'un sel de tramadol et de carraghénane pour former la matière à base de tramadol complexé sous forme d'une pâte, le séchage de la pâte, la formation de granules à partir de celle-ci et le compactage des granules pour former la forme compactée.

**20.** Procédé selon la Revendication 16, comprenant l'utilisation d'un sel de tramadol et de lambda-carraghénane pour former la matière à base de tramadol complexé, la formation de granules à partir de celle-ci, le compactage des granules pour former la forme compactée, et la formation d'une couche supplémentaire par-dessus ladite forme compactée, la couche supplémentaire incluant un agent retardateur polymère hydrophile, de l'acétaminophène et une matière à base de tramadol qui est principalement non complexée.

**21.** Procédé selon la Revendication 20, comprenant l'utilisation d'un rapport molaire compris entre 1:1 et 20:1 ou entre 5:1 et 10:1, de l'acétaminophène sur la matière à base de tramadol pour former la forme compactée.

**22.** Procédé selon la Revendication 20, de sorte que dans la libération prolongée, lorsque la libération cumulative en % massiques de tramadol est de 40 % en masse, la libération cumulative en % massiques d'acétaminophène est différente de moins de 25 % en masse de la libération cumulative en % massiques de tramadol.

**23.** Procédé selon la Revendication 20, où dans la libération prolongée démarrant du point où la libération cumulative en % massiques de tramadol est de 40 % en masse, la libération cumulative en % massiques d'acétaminophène n'est jamais différente de plus de 20 % en masse de la libération cumulative en % massiques de tramadol.

**24.** Procédé selon la Revendication 16, comprenant l'utilisation d'au moins deux types différents d'hydroxypropylcellulose dans la fabrication de la forme compactée.

**25.** Matière à base de tramadol complexé et acétaminophène pour utilisation dans un procédé de traitement de la douleur, où lesdits matière à base de tramadol complexé et acétaminophène présentent une libération prolongée coordonnée desdits tramadol et acétaminophène lors d'une administration orale chez un patient, résultant en une libération cumulative coordonnée de tramadol et une libération cumulative d'acétaminophène au cours du temps, et où la matière à base de tramadol complexé est complexée à l'aide de carraghénane.

## FIG. 1A

## FIG. 1B

## FIG. 1C

## FIG. 2

Release profile of Acetaminophen / Tramadol combo
from a matrix with and without a complex of Tramadol

## FIG. 3

Release profile of Acetaminophen / Tramadol combo
from a matrix (Formulation C)

## FIG. 4

Release profile of Acetaminophen / Tramadol combo
from a matrix (Formulation D)

## FIG. 5

Release profile of Acetaminophen / Tramadol combo
from a matrix (Formulation E)

# FIG. 6

## Effect of HPMC content

# FIG. 7a

## Release profile of Acetaminophen / Tramadol combo from a matrix (Formulation F)

# FIG. 7b

Release profile of Acetaminophen / Tramadol combo
from a matrix without λ-carrageenan (Formulation G)

## FIG. 8

In vitro release for Acetaminophen from different formulations of matrix tablet (F-No. 2-5)

## FIG. 9

In vitro release for Tramadol HCI from different formulations of matrix tablet (F-No. 2-5)

## FIG. 10

Simulated release profiles assuming IR layer content for
Acetaminophen from different formulations of matrix tablet (F-No. 2-5)

## FIG. 11

Simulated release profiles assuming IR layer content for
Tramadol HCl from different formulations of matrix tablet (F-No. 2-5)

## FIG. 12

In vitro release profiles for Acetaminophen
and Tramadol HCl from F-No. 6

## FIG. 13

Simulated release profiles assuming IR layer content for
Acetaminophen and Tramadol HCl from F-No. 6

## FIG. 14

In vitro release profiles for Acetaminophen
from F-No. 7 and F-No. 8

## FIG. 15

In vitro release profiles for Tramadol HCl
from F-No. 7 and F-No. 8

## FIG. 16

Simulated release profiles assuming IR layer content for Acetaminophen from F-No. 7 and F-No. 8

## FIG. 17

Simulated release profiles assuming IR layer content for Tramadol HCl from F-No. 7 and F-No. 8

## FIG. 18

In vitro release profiles for Acetaminophen
from F-No. 7, F-No. 9 and F-No. 10

## FIG. 19

In vitro release profiles for Tramadol HCl
from F-No. 7, F-No. 9 and F-No. 10

# FIG. 20

Simulated release profiles assuming IR layer content for Acetaminophen from F-No. 7, F-No. 9 and F-No. 10

# FIG. 21

Simulated release profiles assuming IR layer content for Tramadol HCl from F-No. 7, F-No. 9 and F-No. 10

## FIG. 22

In vitro release profiles for Acetaminophen
from F-No. 10, F-No. 11 and F-No. 12

## FIG. 23

In vitro release profiles for Tramadol HCl
from F-No. 10, F-No. 11 and F-No. 12

## FIG. 24

Simulated release profiles assuming IR layer content for Acetaminophen from F-No. 10, F-No. 11 and F-No. 12

## FIG. 25

Simulated release profiles assuming IR layer content for Tramadol HCl from F-No. 10, F-No. 11 and F-No. 12

## FIG. 26

In vitro release profiles for Acetaminophen from F-No. 10

## FIG. 27

In vitro release profiles for Tramadol HCl from F-No. 10

## FIG. 28

Simulated release profiles assuming IR layer content
for Acetaminophen from F-No. 10

## FIG. 29

Simulated release profiles assuming IR layer content
for Tramadol HCl from F-No. 10

## FIG. 30

In vitro release profiles for Acetaminophen from
F-No. 7 at 50, 75 and 100 rpm

## FIG. 31

In vitro release profiles for Tramadol HCl from
F-No. 7 at 50, 75 and 100 rpm

## FIG. 32

Simulated release profiles assuming IR layer content for Acetaminophen from F-No. 7 at 50, 75 and 100 rpm

## FIG. 33

Simulated release profiles assuming IR layer content for Tramadol HCl from F-No. 7 at 50, 75 and 100 rpm

# FIG. 34

In vitro release profiles for (a) Acetaminophen and (b) Tramadol HCl from F-No. 13 at 50 rpm in pH 1.2 buffer solution for first 2 hours and pH 6.8 buffer solution from 2 to 12 hours

# FIG. 35

APAP
Tramadol HCl
Powered cellulose
Pregelatinized Starch
Sodium Starch Glycolate

APAP
HPMC E15

| Fluid Bed Granulator/Dryer |

| Super mixer Granulator |

| Preblend |

| Preblend |

| Granulation | ← Corn Starch
Purified water

| Granulation | ← Tramadol HCl
λ-Carrageenan
Purified water
EtOH

| Drying |

| Drying |

Mg. Stearate →

HPMC K4M
Polyox
Mg. Stearate

| Sieving |

| Sieving |

| Blending |

| Blending |

| Compression |

Opadry
Purified water → | Film Coating |
Carnauba wax

| Finished Bulk Product |

## FIG. 36

Mean plasma Tramadol concentration-time profile
(Log scale)

## FIG. 37

Mean plasma Acetaminophen concentration-time profile
(Log scale)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US RE39221 E
- WO 2004026308 A
- US 20040131671 A
- US 3652589 A

### Non-patent literature cited in the description

- **AGUZZI et al.** Influence of Complex solubility on Formulations based on Lambda Carrageenan and Basic Drugs. *AAPS PharmSciTech,* 2002, vol. 3 (3
- **WILDES et al.** *J. Org. Chem.,* 1971, vol. 36, 721
- **AGUZZI et al.** *AAPS PharmSciTech,* 2002, vol. 3 (3
- Dissolution. The United States Pharmacopeia. The United States Pharmacopeial Convention, 2007, 277-284
- Dissolution testing as a prognostic tool for oral drug absorption: immediate release dosage forms. **DRESSMAN, JENNIFER B. ; AMIDON, GORDON L. ; REPPAS, CHRISTOS ; SHAH, VINOD P.** Pharmaceutical Research. Plenum Publishing Corp, 1998, vol. 15, 11-22
- The role of dissolution testing in the regulation of pharmaceuticals: the FDA perspective. **SHAH, VINOD P.** Pharmaceutical Dissolution Testing. Taylor & Francis, 2005, 81-96
- Regulatory perspectives on in vitro (dissolution)/in vivo (bioavailability) correlations. **UPPOOR, V. R. S.** Office of Clinical Pharmacology and Biopharmaceutics, FDA, CDER, Rockville, MD, USA, Journal of Controlled Release. Elsevier Science Ireland Ltd, 2001, vol. 72, 127-132